# EUROPEAN PATENT SPECIFICATION

(11) **EP 1 605 264 B1**
(45) Date of publication and mention of the grant of the patent: **11.01.2012**
(21) Application number: 04717012.1
(22) Date of filing: 04.03.2004
(51) Int. Cl.: B01L 3/00

(54) **AN INTEGRATING ANALYSIS CHIP WITH MINIMIZED REACTORS AND ITS APPLICATION**
INTEGRIERENDER ANALYSECHIP MIT MINIMIERTEN REAKTOREN UND DESSEN ANWENDUNG
PUCE D'ANALYSE INTEGRANT DES REACTEURS MINIMISES ET APPLICATION ASSOCIEE

(30) Priority: 04.03.2003 CN 03117397; 19.06.2003 CN 03135251; 30.09.2003 CN 03135958
(43) Date of publication of application: 14.12.2005
(73) Proprietor: Chengdu Kuachang Medical Industrial Limited, Chengdu, Sichuan 610041 (CN); Chengdu Kuachang Science & Technology Co., Ltd., Sichuan 610041 (CN)
(72) Inventor: ZOU, Fanglin, Chengdu, Sichuan 610000 (CN); CHEN, Chunsheng, Sichuan 610000 (CN); CHEN, Ning, Panzhihua, Sichuan 610000 (CN); WANG, Jianxia, Meishan, Sichuan 610000 (CN)
(74) Representative: Grünecker, Kinkeldey, Stockmair & Schwanhäusser
(86) International application number: PCT/CN2004/000169
(87) International publication number: WO 2004/083863

(56) References cited:
- WO-A-02/052045
- WO-A1-02/052045
- WO-A1-02/090963
- WO-A2-01/02093
- CN-A- 1 273 609
- CN-A- 1 274 758
- CN-A- 1 281 147
- CN-A- 1 314 493
- CN-A- 1 325 026
- CN-A- 1 335 501
- CN-A- 1 343 790
- CN-A- 1 417 348
- CN-A- 1 448 723
- CN-Y- 2 280 908
- US-A- 5 545 531
- US-A- 5 622 872
- US-A1- 2002 009 728
- US-B1- 6 379 929
- CUI DAFU: 'DNA chips research based on bioMEMS technologies' JOURNAL OF MECHANICAL STRENGTH vol. 23, no. 4, 2001, pages 471 - 475, XP008040801

## Description

### Field of the Invention

This invention involves an analysis-chip, a kind with multiple reactors in particular. To be more exact, it is a highly integrated analysis-chip with minimal- height reactor. This invention involves also application of this analysis-chip.

### Background of the invention

Analysis-chip, chip in short, has been applied in various fields, including the determination of gene expression, gene screening, drug screening, diagnosis and treatment, environmental monitoring and management, appraisal of justice, and etc.

The core of chip is the reactor thereon, while the chip with markers also includes marking system. The three most important components of the reactor are: probe array, substrate and reactor structure. Probe array is formed by immobilizing probes used in analysis such as polypeptide, nucleic acid etc onto the substrate surface through spotting, printing etc. Substrate is usually a planar carrier containing active derivative group, which is made of glass, metal, plastics or their derivatives in the shape of rectangle, round or others. At present, the mainstream substrate in use is made chiefly of glass and the derivatives thereof (e.g. amino group plate, aldehyde group plate, epoxy group plate and polyamino acid-coated plate etc.) The reactor structure includes the partition structure, flow-path structure, separation structure, chamber structure, etc.

The rapid development of chip is closely related to its high integration, which involves the following four aspects: device miniaturization, high-density probe array, high-density reactor and integration of multi-functions. In the development of highly-integrated chip, significant progresses have been made in the three aspects: the first is the highly densified probe distribution (e.g. chip with high-density probe); the second is multi-functioned devices (e.g. chip-on-lab); the third is device miniaturization. However, with enhanced practical applications, new problems have emerged in the development of chip. For example, in polypeptide chip it is usually unnecessary to immobilize different types of probes, then what is the significance of high density? Another example is: in extensive diagnosis and screening, test device at low cost is needed, then how could multi-function be achieved at low cost? The inventors of this invention hold that the chip with high-integration and low cost remains one of important aspects of chip development.

The current situation in chipdevelopment:

### 1. Chip and reactor partition structure

The foremost-developed chip, which is still in wide use today, is a chip with no partition structure (e.g. open non-flow chip with single reactor based on microscope slide, made through activation and spotting, without other additional structures. advantages are: the structure is simple; spotting and scanning is easy and convenient; some processes can be performed while the media is in flowing status propelled by exterior forces (e.g. liquid spray for washing). Its disadvantage is: only one probe array is immobilized on the chip. When fewer types of targets are to be detected in a sample, e.g., less than 100 types, and consequently not many types of probes are need to be fixed on every reactor, it is usually the ratio between probe region on substrate of reactor and the average substrate area on reactor that decides the cost of chip. Therefore, in order to reduce cost, it is required to develop the chip with high-density reactor. However, in the development of chip with high-density reactor, one of main problems to be solved is the selection of appropriate reactor partition structure. In addition, in order to reduce sample volume, the flow of liquid media also has to be controlled. Moreover, the thickness of reactor partition structure has to be restricted on account of the wide applications of optical scanning equipment. So, the partition structure with a height close to zero will make small technical requirements on scanning equipment. Meanwhile, the minimization of partition structure in height can also faciliate washing.

In order to increase benefit, manufacturers and researchers have made improvements on chips with no partition structure. As a result, chips with partition structure have been developed, which can be divided into two types: the detachable and the undetachable. Now a type of chip with undetachable partition structure available in market is a chip with multi-open non-flow reactors. Its basic structure is : upon a standard baseplate with a size of 25mm x 75mm or 26mm x 76mm (width x lengthheight), hydrophobic materials (surface contact angle is 50-75°, e.g., polyester or PVC) are used to fabricate a convex with a height of more than 0.5mm, and a width of over 1 mm. Thereby a few to scores of round or square open reactors are formed on baseplate. So, in this chip, the flow of liquid media is still controlled on the basis of the difference in height of partition structure. In this type of chip, if the partition area among open reactors is too low, the cross-contamination with neighbor reactors will occur; while if it is too high, it will make a higher technical requirement on biochip scanner. Besides, another type of chip available at present is based on the structure of ELISA plate with detachable partition structure, whose main drawbacks lie in the complicated operation or the height of partition structure among reactors, which cannot be minimal.

So, despite various designs of partition structure(e.g. No.02145102.8 and 99114512.7 China Patent Application), how to develop an easily manufactured partition structure with minimal height at low cost remains one of concerned subjects.

### 2. Chip and reactor flow-path structure, chip and reactor separation structure

At present, microflow-path, or microchannel is the chief flow-path structure and separation structure widely studied. The microchannel usually is of less than 0.10mm in width and less than 0.025mm in depth. So the current microflow-path is actually a concave flow-path.

The chip with microflow-path is called microchannel chip; that is a comprehensive analysis system in mininature with sampling, pre-processing, liquid-transporting and other analytic functions rolled into one, where microflow-path serves as the network to connect micropump, microvalve, microvessel, microelectrode, microdetection units etc. The example for microchannel chip is the analysis-chip of Caliper Technologies Inc. (www.caliper.com). The advantages of microchannel biochip are high sensitivity and fast speed, while the disadvantages are: 1) The microchannel has to be etched beforehand. Then after probe is deposited thereon, the microchannel has to be sealed. With the complicated structure, industrialization is much difficult; 2) In the detection, the flow speed of liquid has to be controlled by some sophisticated instruments, e.g. electronic osmosis device and etc.; 3) after reaction, because probe molecules are fixed on the innner surface, for some detections like fluorescence- labeled detection, the test results cannot be read directly with a common biochip scanner. Despite various projects on microflow-path recently (e.g. USA Patent No. 6176962 and 6180536), how to develop an easily manufactured flow-path structure at low cost remains one of concerned subjects.

### 3. Chip and the structure of the reaction chamber

Reactor includes open reaction well and closed reaction chamber. The chip with reaction chamber is a closed chip, though parts of partition structure are still open. Closed chip includes flowing and non-flowing chips. There is a kind of closed non-flowing chip, during reaction reactor is closed, while washing and cleaning, seal layer is disclosed (refers to CN 1335501A). For the closed microarray flow chip, it can be refered to China Patent ZL 022229310. For the irreversible closed microarray flow chip, it can be refered to CN 2559986Y. For the reversible closed microarray flow chip, it can be refered to China Patent ZL 022229310, etc. Owing to ligand is fixed only on one surface of reaction chamber of the chips; it is difficult to satisfy the requirements for especially higher sensitivity. Particularly, for the existing flow chips with closed chamber, the dynamic conditions for ligand-target reaction and conditions for observing signal are the factors concerned while designing. The movement of liquid phase media in the reactor, especially in the reaction chamber of reactor, is completed through mechanic transport, the weight of liquid media itself, and the hydrophilicities of liquid-inlet structure or/and outlet structure or more than one type of combinations. For reaction chamber, all of these are exogenous liquid-delivering impetus. Of course, the top plane, bottom plane and wall of reaction chamber usually possess hydrophilicity; but this kind of endogenous liquid-delivering impetus of hydrophilicity only is weak. Consequently, on recent closed microarray chip, while running a test, especially performing the operations of media changing, sometimes the distribution of liquid media in the reaction chamber of reactor is insufficient. For example, when gas exists, it will inhibit the distribution of liquid media, thereby interfering test result. At present, there is one kind of capillary chip, wherein the reaction chamber is fabricated by immobilizing ligand onto a length of wall of capillary passage or a length of glass fiber structure which is inserted into the capillary (CN 2483395A). But, the inner wall of capillary or the structure of glass fiber, which could be inserted into capillary, is very slim, so it is unsuitable for manufacturing array chip. Besides, it is not allowed when using some signal-reading equipments (e.g. laser con-focal scanner) for reading signal directly under the condition of the existence of chamber top unit in the reaction chamber.

### 4. Chip and marking system

As for current chips, one type requires pre-deposited markers in the reactor e.g. China Patent (A low-density biochip and the preparing method thereof, Patent Application No. 00129440.7), which will release instantly in testing, while the other type requires that the marker solution be subjected to sample or to the reactor after probe-target reaction is completed. The latter marking system, though of higher sensitivity, is difficult to operate. The former marking system is easier to operate, however, owing to Hook effect etc., its test sensitivity is greatly decreased.

When this type of marking system is adopted in chip analysis, to avoid Hook effect, all the markers should be in liquid status, which, if needed, is subjected into reaction device completely through mechanic system. For example, when planar chip with outlaying marking system and channel chip with inlaying marking system to are employed in analysis, special marker solution loading procedure and corresponding mechanic system are required. In other words, in the present chip analysis, since the labeling procedure is to be carried out with mechanic system which helps deliver markers, special marker container. marker-delivering channel, converter for the delivered material and conversion procedures are usually indispensable, thus making the test device complicated. Besides, since only small amount of marker is needed in the detection, the storage and preparation of markers in both marking systems are much troublesome. Though the present microchannel chip is equipped with inlaying marking system, markers are released instantly in detection, therefore special procedure for adding marker solution and corresponding mechanic system are required.

### 5. Reactor-protecting system

As chip with high-density reactors is introduced, one important but often neglected problem in its applications is: if only M out of N reactors (N>M) are used in one testing, how to preserve other N-M reactors for later use. The solution to this problem serves as prerequisite for developing chip with high-density reactors at low-cost. As for chip with single reactor, the reactor-protecting system at present is actually a protecting system for whole chip, which usually is a plastic box containing chip rack (grid). But strangely, the chip with multi-reactors now in use is equipped with no special protecting structure but the said protecting plastic box for the chip with single reactor.

Thus, the chip constituted by present reactor partition structure, reactor flow-path structure, reactor separation structure, marking system and reactor-protecting system are yet to be further improved.

### SUMMARY OF THE INVENTION

The purpose of this invention is to provide a highly integrated analysis-chip and the corresponding devices, which should be of easy preparation, lower cost, high sensitivity, small sample consumption, simple operation, and homogeneous distribution of reaction media.

In the course of developing biochip for blood screening, tumor marker detection, and hepatitis screening, in order to render the researched chips to have more competitiveness at cost besides better quality than recently used ELISA reagent kit, we realized that to improve the utility of substrate, one of factors for controlling the cost of chip, or in other words, to improve integration of the chip is one of the keys to the problem. In addition, we also found that it is very important to simplify operation procedures, facilitate washing, lower the height of reactor structure and reduce the investment on scanner by lowering the technical requirements on it in detection. Hence, "minimizing the height of reactor structure and maximizing the integration level" become our important research subjects, which results in this invention.

The goal of this invention is fulfilled through following fashions:
the first embodiment of this invention provides an analysis-chip, which comprises one or more reactors with minimal height, wherein said reactor comprises at least: 1), one or more striped-capillary reaction-chambers, wherein said reaction-chamber comprises: i). top plane and bottom plane with a width more than 600um; ii). probe-ligand and substrate probe-region in which said probe-ligand is immobilized; iii).closed partition-structure with a height of 1-1000 µm, optimally 1-500µm; and iv).inlet and outlet, 2). reactor structure with minimal-height; which comprises at least open partition-structure comprising: i). substrate blank region with a width of 0.5-10mm; or/and ii)one or more following convexes with a height less than 1000µm, optimally less than 500µm: hydrophobic convex, highly-hydrophobic convex, and water-absorbing convex; and optionally, iii). convex flow-path comprising high-hydrophilic convex with a width of 5-4000µm, and a height of 0.05-1000µm, optimally 0.05-500µm relative to said substrate probe region; and optionally, reactor-protecting structure comprising protective unit with a distance less than 1000µm, optimally less than 500µm from said substrate probe-region; and optionally, 3). marking-system convex, a marker-containing convex that does not cover said probe-ligand, wherein: (1). said top plane and bottom plane are parts of top unit and bottom unit of said striped-capillary reaction-chamber, respectively; (2). said substrate probe-region is on said top plane or/and bottom plane; (3).said closed partition-structure is placed between said top plane and bottom plane; (4). material and dimension of said planes, and distance between said top plane and bottom plane are such that the capillary phenomenon of analysis media can take place in said striped-capillary reaction-chamber; (5).said substrate blank region and substrate probe region are on the same plane of a same substrate, which presents a water-absorptivity less than 0.1 g/g and a water contact angle as 40-80°;(6).said highly-hydrophobic convex contains, at least in its partial surface, highly-hydrophobic material, wherein said highly-hydrophobic material presents a water contact angle of 40° bigger than that of said substrate probe region; (7). said hydrophobic convex contains, at least in its partial surface, hydrophobic material, wherein said hydrophobic material presents a water contact angle as 55-80° ;(8). said water-absorbing convex contains, at least in its partial surface, water-absorbing material, wherein said water-absorbing material presents a water-absorptivity more than 0.1g/g; (9). said highly-hydrophilic convex contains, at least in its partial surface, highly-hydrophilic material, wherein said highly-hydrophilic material presents a water contact angle less than 40°; and (10). said protective unit closes at least partially said reactor structure when no sample is subjected, and it is irreversibly removed completely or partially when sample is to be subjected.

The striped-capillary reaction-chamber of analysis-chip of the first or second embodiments of this invention differs from the reaction-chamber of the present closed-flow-chip: the former is characterized by its requirement on the selection of material and dimension of said planes, and distance between said top plane and bottom plane, so that the capillary phenomenon of analysis media can take place in said striped-capillary reaction-chamber. Whereas the latter lacks such requirements which make it necessary to introduce other media distribution mechanism, such as putting a membrane in the chamber etc.

The striped-capillary reaction-chamber of this invention differs from the reaction-chamber of the present micro-channeled analysis-chip: 1). the former presents wider substrate probe region (>500µm) for immobilizing bigger probe array, whereas the latter narrow substrate probe plane (<500µm); 2) the former can apply the capillarity with much bigger flow for direct media distribution; but the latter applies the capillarity with only tiny flow, which requires additional energy for media distribution.

The striped-capillary reaction-chamber of this invention differs from the present capillary analysis-chip: the capillary structure of the former is formed by two planes as well as the structure between the two planes, which make it possible to fix a bigger probe array there, whereas the latter couldn't.

The top plane and bottom plane of the striped-capillary reaction-chamber of the analysis-chip of this invention could be in various geometric figures like rectangular, round etc. The materials of the top plane or/and bottom plane, especially the material for the substrate probe region could be selected from one of the following or their optional combinations: glass, silicon and silicide, metal oxide, metals and polymer materials as well as their derivatives etc. The probe array can be in various ordered distributions like matrix, broken lines, real lines etc. In fact, according to the various applications of chips, it can have and must have various selections of ordered ligand-distribution patterns. Said probes can be selected from one material of the following groups or their optional combinations: antigens, antibodies, ligand, aptamer screened by Systematic Evolution of Ligands by Exponential Enrichment (SELEX), polypeptides, single-strand or multi-strand DNA, nucleotides, polynucleotides, saccharides, coenzymes, cofactors, antibiotics, steroids, viruses and cells.

The convex flow path of the invention especially differs from the microflow path of present chips. The flow path of the latter needs to make channel, trough or capping, and is a concave flow path, its manufacturing is complicated and its cost is expensive.

This invented convex of marking system can be in various geometric figures, such as dot, line, strip, sphere etc.
In this invented chip, the optimized pattern for said striped-capillary reaction-chamber is: the closed partition-structure presents a height of 1-300µm, optimally 30-100µm; The top plane presents a width of 1000-15000µm and a length more than 1000µm; and the bottom plane presents a width of 1000-15000µm and a length more than 1000µm.
In the striped-capillary reaction-chamber, the widths of liquid-inlet and liquid -outlet are equal to or not equal to the widths of the top plane and bottom plane, and the heights are equal to or not equal to the space between said top plane and bottom plane. We discovered, in the situation of utilizing capillarity to distribute media directly, it is necessary to maintain certain height (e.g. 30um) of chamber. Besides, we also discovered, when using mechanic force to distribute reaction media, to maintain certain height of chamber is beneficial for avoiding uneven distributions, such as air bubble, etc.

In this invented chip, the optimized pattern for the striped-capillary reaction-chamber is: the widths of the top plane and bottom plane are 1500-15000mm, preferably 2500-15000mm.

In this invented chip, said closed partition structure includes one or more of the following reversible or irreversible enclosing structures:
1). thermal enclosing structure;
2). chemical enclosing structure;
3). reversible or irreversible adhesive layer;
4). highly-hydrophobic layer;
and 5). mechanic enclosing unit including coating, plate or tape of elastic polymer.

Said closure mainly refers to the closure of borders between top unit and bottom unit except liquid-inlet and liquid-outlet. The closure mainly is the irreversible closure generated by the ways including adhesion or/and heat-fusion, or/and HF or sodium silicate linkage at low temperature. The closure of adhesion can be irreversible closure or reversible closure. The closure formed by highly-hydrophobic water-partition seal and mechanic seal mainly is reversible closure. For irreversible closure, when necessary, the top plane or bottom plane can be opened by mechanical action to open the chamber cover. Said mechanical seal units all possess a certain elasticity, for example, their Shore hardness are between 20-100°. and are made from rubber (e.g. natural rubber and the derivatives thereof: synthetic rubbers like acrylonitrile-butadiene rubber, butyl rubber, fluororubber, silicone rubber, fluorosilicone rubber etc.) and various organic polymers (e.g. organosilicon polymer, fluoropolymer, polycarbonate, plastic etc.).
In this invented chip, the top unit or/and bottom unit of said reaction-chamber present a thickness less than 1mm, optimally less than 0.2mm, and a detecting-light transparency rate more than 90%. In this invention, the example for the transparent unit is a cover glass or activated cover glass with a height of 0.15mm. So, the test result on the invented chip can be visualized directly with signal-testing instrument. Said signal-testing instrument includes laser confocal scanner and laser scanner. In the implementation examples, when the cover glass of the top unit is thin enough (e.g. ≤ 0.15mm), the invented chip, unnecessary to be dismantled, can be used directly for the scanning with scanner, showing no obvious differences of the results between using a cover glass or not.

In this invented chip, said probe-ligand is immobilized on either said top plane or said bottom plane,
wherein said plane with said immobilized probe-ligand presents hydrophilic property whereas said plane without said immobilized probe-ligand presents hydrophobic property. The top plane is made of glass material whereas said bottom plane is made of hydrophilic or hydrophobic plastic
In this invented chip, one or more said probe-ligands are immobilized in one area whereas one or more ligats of said ligands are immobilized in one other area in said probe region. Said ligand and ligate include antigen and antibody, such as antibody and anti-antibody which are corresponding to said antigen etc. So this invented chip provides a new tool with high sensitivity, which can be used for detecting antigen and corresponding antibody simultaneously,
In this invented chip, said convex flow-path refers to a coating with said highly-hydrophilic material. When necessary, said convex flow-path comprises hydrophobic convex or/and highly-hydrophobic convex as its partition structure, wherein said convex presents a height less than 1000um, optimally less than 500um.

In this invented chip, said convex flow path can has separation media immobilized. Said separation media include electrophoresis media or chromatography media.

said highly-hydrophobic convex, hydrophobic convex, highly-hydrophilic convex, water-absorbing convex are made respectively by solidifying liquid material onto the surface of the chip, or/and immobilizing solid substance onto the surface of the chip. Said liquid materials include solution, paint, gel or emulsion containing highly-hydrophobic material, hydrophobic material, highly hydrophilic material, and water-absorbing material respectively. Said solid substances include plate, film, board, strip or powder containing highly-hydrophobic material, hydrophobic material, highly hydrophilic material, and water-absorbing material respectively.
The following example indicates how said convex is prepared by solidifying liquid material: A. providing the liquid material that has affinity to substrate; B. coating it onto the specified position on the substrate; C. solidifying the coating by drying or/and adding solidifying agent, wherein the solidifying reactions include oxidation polymerization (e.g. in case of the modified paint with drying oil etc.), solvent volatilization (e.g. in case of varnish dried and solidified at normal temperature etc.), solidification by using solidifying agent, solidification by heating, catalysis by bio-enzyme (e.g. nature lacquer etc.) and so on.
The following example indicates how said convex is prepared by immobilizing solid material: A. providing the shaped solid substance which can bind to substrate; B. binding the solid substance onto the specified position on the substrate by adhesion or/and thermal fusion.

In this invented chip, the height of said highly-hydrophobic convex is optimally 0.1-100µm. In fact, in the implementation examples, when its height is 20-50µm, desirable results can be obtained.

In our research, we found that hydrophobicity, as a kind of reactor partition mechanism, can effectively confine water solution to the surface of hydrophilic substrate surrounded by highly-hydrophobic material. Table 1 reveals the effects of some highly-hydrophobic coatings:

**Table 1 partition effects of some highly-hydrophobic coatings**

| Highly-hydrophobic paint | Static water contact angle | The width of coating | The thickness of coating | Surface area of hydrophilic substrate | The loading quantity of water | Down flow at 90° |
|---|---|---|---|---|---|---|
| Organosilicon paint | 95" | 1mm | 30-40µm | 16mm² | 20µl | |
| Nano-textile | 155° | 1mm | 400µm | 16mm² | 30µl | - |
| Nanometer silica CDJ7 | 160° | 1mm | 20-30µm | 16mm² | 30µl | - |
| Black paint (ChuangKePeng) | 85° | 1mm | <5µm | 16mm² | 15 µl | - |
| Substrate without coating | 45° | - | - | 16mm² | 10µl | + |

In the table, down-flow at 90° means making the chip vertical to the horizontal plane then observing whether there is the flow of water. When water does flow downward, the result is positive (+), while when there is no water flow, the result is negative (-).

In this invented chip, said highly-hydrophobic material presents a water contact angle that is over 70° bigger than that of said substrate probe region, preferably over 90°, especially preferably over 110°.

In this invented chip, said highly-hydrophobic material includes highly-hydrophobic organic material or/and highly-hydrophobic nano-material. said highly-hydrophobic material includes one or more of the following materials:
1). highly-hydrophobic organosilicon and its derivatives;
2). highly-hydrophobic fluororesin and its derivatives;
3). highly-hydrophobic polymer; and
4). paint or/and solid substance containing highly-hydrophobic nano-particle.
Said organosilicon materials are organosilicon polymer, copolymer based on organo-siloxane bond, or the highly-hydrophobic derivatives thereof. Said fluororesins include: fluororesin oligomer, polytetrafluoroethylene (PTFE), copolymer fluororesin (PFEP), polytrifluorochloroethylene, polypolyvinylidene fluoride. Said highly-hydrophobic polymers include highly-hydrophobic polypropylene, polyacrylate and the derivatives thereof. In the coating or/and solid substance with said highly-hydrophobic nanoparticle. said nanoparticles include one or more of the following nanoparticles with a diameter of 1nm-100nm: gold, vanadium, lead, silver, iron and oxide powders thereof, silica, titanic oxide, alumina powders and the derivatives thereof.

In this invented chip, said hydrophobic convex includes hydrophobic coating. said hydrophobic coating includes colored hydrophobic line, or/and colored hydrophobic strip.

In this invented chip, said highly-hydrophobic material presents a surface static water contact angle of less than 30°. Said highly hydrophilic materials include highly hydrophilic nanometer material (e.g. highly hydrophilic nanometer material) and highly hydrophilic polymer material (e.g. highly hydrophilic polyacrylate acid paint).

As one of the designs for invented ananysis-chips, said water-absorbing material includes one or more of the following:
1). natural water-absorbing material;
2). solid porous material of hydrophilic inorganic compound; and
3). water-absorbing material of synthetic polymer.
said water-absorbing material includes one or more of the following: a). capillary-structure-containing paper product, cotton product, or/and sponge as well as their modified materials, b). calcium salt; c). water-absorbing materials based on cellulose or its derivative, d). water-absorbing materials based on starch or its derivative, e). water-absorbing materials based on synthetic resin as well as compound generated by grafting, blocking and copolymerizing, paper product, cotton product, sponge and its modifier, calcium salt, water-absorbing cellulose material, water-absorbing starch material, and water-absorbing synthetic resin produced by grafting, blocking and copolymerizing.
Said marking-system convex refers to convex of controlled marker-releasing system, wherein said controlled marker-releasing system comprises marker and presents a half-releasing-period of said marker of more than 10 seconds, optimally more than 30 seconds, wherein said marker comprises labeling reagent and marking-ligand,

The example for controlled release marking system is the programmable release system, in which the release velocity and the lag-time of marker are defined mainly by the compositive material and structure of system itself. For the marker in said convex of marking system, the labeling methods include chemiluminescent labeling, excitation luminescent labeling, non-selective light reflection labeling and selective light reflection labeling. Wherein 1). said labeling reagent includes one or more of the following reagents: enzyme, fluorescent dyestuff, chemiluminescent catalyst, nonferrous metal or nonferrous metallic salt, dyestuff and paint;
and 2). said ligand includes one or more of the following substances: antigen, antibody, biotin, drug ligand polypeptides, DNA, RNA and the fragments thereof.
In this invention, the term "half-releasing-time" refers to the time needed to release 50% of markers from controlled marker-releasing system into reactor under the condition of labeling reaction. Obviously, this invented marking system is a controlled marker-releasing system. The convex with controlled marker-releasing system can be of various technical designs, for instance, it can be made into a sphere or other solid figures containing liquid or solid markers and the release retarder; or a dot or line etc. with markers and release retarder. In these cases, the marking convex presents a height less than 1000µm, and is fixed around the array of said probe-ligand in said reactor or inside the array of said probe-ligand to form an array of probe-ligand and marking system.

As one of designs for the invented chips, said controlled marker-releasing system comprises said marker and controlled-releasing agent.

The release retarder described in this invention refers to the materials that can controll or take part in controlling the releasing speed of markers and delay the releasing, such as the diffusion-controlling agent that controls the release of markers by controlling the density of the controlling agent in the network; the chemical reaction-controlling agent that controls the release by controlling degradation; solvent activation-controlled release agent that controls the release by controlling the activation process (e.g. dissolving); the release retarders that speeds up release by using water-absorbing and swelling materials inside membrane to make membrane dilate and disintegrate and etc.

With diffusion-controlling system, the markers are released according to the fluctuation of density of diffusion-controlling agent (e.g. polymer with moderate solvency and dilatability) in the network; with chemical reaction-controlling system, markers are released when the controlling agent (e.g. degradable polymer) is degraded; with activation-controlling system, markers are released when the controlling agent (e.g. membrane) is activated (e.g. dissolved) by solvent.

For example, in diffusion-controlling system with markers evenly dispersed or embedded in polymer with moderate water-solubility (release retarder), the releasing speed of markers is decided by the dissolving and swelling speed of polymer, thereby the concentration of marker can be adjusted to accommodate the requirement in different testing procedures.

For another example, in chemical reaction-controlling system with markers evenly dispersed or embedded in the degradable polymer (release retarder), the marker-releasing velocity is actually zero when sample is subjected. While labeling, markers are released due to the degradation of polymeter with the help of degrading agent (e.g. enzyme). For still another example, in activation-controlling system made up of markers and membrane (release retarder), the release or delayed release of marker is decided by the speed of membrane dissolution and dilation.

As one of designs for the invented chips, said convex of controlled marker-releasing system includes mono-sandwiched or multi-sandwiched structure made up of said marker and controlled-releasing agent, wherein said sandwiched structure refers to structure where the concentration of said marker is higher inside than outside.

In multi-sandwich structure, one or more types of markers and one or more types of release retarders could be comprised, such as, the first layer may comprise marker; the second layer may comprise the structure of enhancing agent.
As one of designs for the invented chips, said controlled-releasing agent includes water-soluble organic compound or organic compound that will disintegrate in water solution. said organic compound includes one or more of the following materials: carbohydrate and its derivatives thereof, plant starch and modified starch, plant glue, animal glue, modified cellulose, polymer and condensate.
As one of the designs for the invented analysis-chips, said protecting unit includes one or more of the followings: organic film or/and plate, film or/and plate of metal-organic complex, and slide.
Said protecting unit is connected with the reactor through one or more of the following reversible/ irreversible enclosing structures: thermal enclosing structure, chemical enclosing structure and reversible or irreversible adhesive layer.

As one of designs for the invented chips, said protecting unit is precut for the convenience of possible removal.

According to another aspect of the invention, it provides an analysis-chip, which comprises one or more reactors with minimal-height,
wherein said reactor comprises probe-ligand, substrate probe region, open partition-structure with minimal height, and optionally, reactor-protecting structure, wherein said open partition-structure comprises:
1). substrate blank region with a width of 0.5-10mm;
2). highly-hydrophobic convex or/and water-absorbing convex, all of which presents a height less than 1000µm, optimally less than 500µm relative to said substrate probe region, wherein:
   (1). said substrate blank region and substrate probe region are on the same plane of a same substrate, which present a water contact angle as 40-80° ;
   (2). said highly-hydrophobic convex contains, at least in its partial surface, highly-hydrophobic material, wherein said highly-hydrophobic material presents a water contact angle of 40° bigger than that of said substrate probe region;
   and (3). said water-absorbing convex contains, at least in its partial surface, water-absorbing material, wherein said water-absorbing material presents a water-absorptivity more than 0.1 g/g.

In fact, the analysis-chips include the analysis-chip with highly-hydrophobic partition structure, the analysis-chip with water-absorbing partition structure and the analysis-chip with highly-hydrophobic/water-absorbing partition structure of the invention. The invented partition structure can be applied into all types of chips, such as the chip in which the smallest distance among reacting well is over 20% longer than the width of reacting well on its outstretched line; the chip with feeding structure or/and liquid-out structure; the chip with one or more structures controlling the directional flow speed of reaction media on the feeding area or/and liquid-out area of the reactor; double-plane chip, and etc. The invented partition structure contains clear area(blank region on substrate with a width bigger than 0.2mm, located on the same plane of probe region on substrate, which is very important and indispensable for chips. Differing from the devices like ELISA plate immobilized with one type of probe, the reactors of the invented chip are usually immobilized with the arrays of varied types of probes therein, demanding the uniformity of the reaction circumstances for every probe in the array. But the existence of highly-hydrophobic convex or water-absorbing convex or highly-hydrophobic/water-absorbing complex convex will change the reaction conditions in its peripheral width defined districts. So, a clear area(blank area between probe region on substrate and convex is required and this area isn't immobilized with efficacious probes. Through numerous experiments, we discovered that the width of the clear area(blank area depends mainly on the height of convex and the surface static water contact angle of highly-hydrophobic material or the absorptivity of water-absorbing material. The convex herein can forms various geometrical figures on the surface of substrate, such as single line, multi-line, strip, lattice and etc. The example for highly-hydrophobic/water-absorbing complex convex is: the highly-hydrophobic convex in the place near to probe region on substrate; alternatively, the water-absorbing convex in the place far to probe region on substrate. In this chip, said reactor is an open-type reactor, in which the highly-hydrophobic convex, water-absorbing convex contained in the open partition structure and protection structure are the same as the descriptions above.

According to another aspect of the invention, it provides an analysis-chip, which comprises one or more open non-flow reactors with minimal-height,
wherein said reactor comprises at least probe-ligand, substrate probe region, open partition-structure with minimal height, and optionally, reactor-protecting structure, wherein said open partition-structure comprises:
1). substrate blank region with a width of 0.5-10mm;
   and optionally, 2).hydrophobic convex with a height less than 1000µm, optimally less than 500µm relative to said substrate probe region,
   wherein:
   (1). said substrate blank region and substrate probe region are on the same plane of a same substrate, which presents a water-absorptivity less than 0.1 g/g and a water contact angle as 40-80°;
   and (2). said hydrophobic convex contains, at least on its partial surface, hydrophobic material, wherein said hydrophobic material presents a water contact angle of 55-80°.

In fact, this analysis-chip is the analysis-chip with clear area(blank partition structure of the invention. Contrary to the visions from many people, through repeated experiments, we discovered amazingly, even if there isn't partition convex, when there is the clear area(blank region on substrate with a width of 1-10mm, under optimal loading amount (e.g. 10um is subjected on 16mm²) and reactive conditions (e.g. 30 minutes at 37°C), no cross-contaminations occurs while reaction. The residual reactant can be absorbed by using water-absorbing material or pipette and even can be washed out directly, no cross-contaminations occur. In the analysis-chip with blank partition structure of the invention, the optional hydrophobic convex is not the main part, but usually is employed as the marker line or/and partition auxiliary. In this chip, said reactor is an open-type reactor, in which highly-hydrophobic convex, water-absorbing convex and protection structure contained in the open partition structure are the same as the descriptions above.
According to another aspect of the invention, it provides an analysis-chip, which comprises one or more flow or non-flow reactors with minimal-height,
wherein said reactor comprises at least one or more striped-capillary reaction-chambers with a height of less than 1000µm, optimally 500µm, and optionally, reactor-protecting structure,
wherein said reaction-chamber comprises:
1). top plane and bottom plane with a width more than 600µm;
2). probe-ligand and substrate probe-region in which said probe-ligand is immobilized;
3). closed partition-structure with a height of 1-1000µm, optimally 1-500µm; and
4). inlet and outlet,
wherin:
(1).said top plane and bottom plane are parts of top unit and bottom unit of said striped-capillary reaction-chamber, respectively;
(2). said substrate probe-region is on said top plane or/and bottom plane;
(3).said closed partition-structure is placed between said top plane and bottom plane;
(4). material and dimension of said planes, and distance between said top plane and bottom plane are such that the capillary phenomenon of analysis media can take place in said striped-capillary reaction-chamber;
and (5). said substrate probe-region presents a water-absorptivity less than 0.1 g/g and a water contact angle as 40-80°. Actually, this type of analysis-chip is the analysis-chip with striped-capillary reaction-chamber of the invention. Said reactor is flowing or non-flowing reactor. The situation of probe immobilization includes three patterns: probe is immobilized on the substrate in bottom unit, probe is immobilized on the substrate in top unit, probes are immobilized respectively on the substrate in bottom unit and top unit. The positions of liquid-inlet and -outlet at least includes three patterns too: they are on bottom unit, on top unit, and in between bottom unit and top unit. The differences of the analysis-chip with striped-capillary reaction-chamber in the invention from present closed flowing chip, microchannel chip, and capillary chip can be referred to the differences between striped-capillary reaction-chamber in this invention mentioned-above and the reactors of present chips.

In this chip, the striped-capillary reaction-chamber and protection structure are the same as the descriptions above. According to another aspect of the invention, it provides an analysis-chip, which comprises one or more flow reactors with minimal-height, wherein said reactor comprises at least:
1). probe-ligand,
2). substrate probe region with a water-absorptivity less than 0.1g/g and a water contact angle as 40-80° ;
3). convex flow-path comprising high-hydrophilic convex with a width of 5-4000µm and a height of 0.05-1000µm, optimally 0.05-500µm relative to said substrate probe region;
and optionally, 4). reactor-protecting structure,
wherein said high-hydrophilic convex contains, at least in its partial surface, highly-hydrophilic material, wherein said highly-hydrophilic material presents a water contact angle less than 40°.

Actually, this analysis-chip is the analysis-chip with convex flow path in the invention, whose convex flow path can be connected to one or more following structures: sample well, reagent well, test well, waste liquid well, separator and etc. Said reactor is flowing or non-flowing reactor. The differences of the invented analysis-chip with convex flow path from present microchannel analysis-chip can be referred to those between the invented convex flow path described above and present microchannel. Similarly, its convex flow path and protection structure are the same as the descriptions above.

In this chip, said reactor is an open reactor. The reactor includes open partition structure mentioned-above.
According to another aspect of the invention, it provides an analysis-chip, which comprises one or more reactors with minimal-height,
wherein said reactor comprises at least probe-ligand, substrate probe region, marking-system convex, and optionally, reactor-protecting structure,
wherein said marking-system convex refers to marker-containing convex that does not cover said probe-ligand. Actually, this analysis-chip is the analysis-chip with immobilized marker in the invention. In the chip, said reactor is an open reactor. And its convex of marking system and protection structure are the same as the descriptions above.

One of the invented analysis-chip, which comprises one or more reactors with minimal height, wherein said reactor comprises:
1). easily-detachable substrate easily dismantled if needed, or/and
2).reactor-protecting structure with minimal-height, comprising protective unit, wherein:
   i). Said height, a distance from substrate probe-region to bottom plane of said protective unit is less than 1000µm, optimally less than 500µm;
and ii). said protective unit closes at least partially said reactor structure when no sample is subjected, and it is irreversibly removed completely or partially when sample is to be subjected. Actually, this analysis-chip is an analysis-chip for selective use in this invention. which includs two kinds: the detachable chip and the openable chip. In the former, substrate can be easily dismantled (e.g. the activated glass, metal, plastic plate and etc. which have undergone the processes of pre-cutting lines on it). In the latter, reactor is covered by sealing unit (e.g. the plastic plate with a removeable area made by mechanic cutting, aluminous plastic film, and plastic film with or without removeable parts, etc.). The protecting structure of the chip is the same as what is described above.

The analysis-chip with a high density of reactors, wherein:
1). said density of reactors is more than 2 reactors/cm² , optimally more than 3 reactors/cm² on at least one plane of a substrate; and
2). said reactor comprises partition structure with a height less than 1 000µm, optimally less than 500µm.

As one of designs for the invented chips, said reactor density is more than 5 reactors/cm² . As one of designs for the invented chips, said reactor is any reactor in claim 1-30, which has a partition structure at a minimal height.

This invention also involves top unit or bottom unit used in above chips. The top unit or bottom unit includes the probe region on substrate and the immobilized probe therein, such as the top unit or bottom unit for generating the striped-capillary reaction-chamber. Said units contain the probe region on substrate and probe and partial or whole chamber walls, partial or whole liquid-inlet and liquid -outlet, partial or whole enclosing structure (e.g. elastic coating and wetness-proof coating), and other structures of the chip (e.g. fixing structure). The material of the top unit is selected from one or optional two or more than two combinations below: glass, silicon, metal oxide, metal and polymer material and their respective derivatives.

This invention also involves a base-plate, comprising:
1). more than one of substrate probe regions; and
2). one or more of the following reactor structures:
   According to another aspect of the invention, it is to provide a qualitative or/and quantitative analysis method, comprising:
   (a) subjecting the sample into the open reactor in an analysis-chip and making the reaction therein; and
   (b) after the reaction is completed, the residual sample needn't cleaned out from the reactor and the followed procedures are carried on directly, said followed procedures include washing or labeling. In this method, the open chip is the open multi-reactors-chip described above.

According to another aspect of the invention, it provides a qualitative or/and quantitative analysis method, including:
(a) subjecting the sample into the reactor of analysis-chip and taking probe-selective reaction and labeling reaction therein at the same time;
(b) washing the reactor and analyzing the result of the reaction. In this method, the chip is the chip described above.

According to another aspect of the invention, it provides a device for detection, said device comprising the pieces for cleaning up the residual in the reactor after the reaction is finished, said pieces performing said cleaning though absorption with water-absorbing material, or/and through washing directly on the chip with an included angle with horizontal plane, said water-absorbing material presents a water-absorptivity larger than 0.5g/g; said included angle is bigger than 5°.

The advantages of the invented highly-integrated analysis-chip are: simple and easy preparation, lower cost, reliable partition, even distribution of media, small consumption of reaction media, high sensitivity, convenient performance and etc.

The advantages of the invented analysis-chip with highly-hydrophobic partition structure, analysis-chip with water-absorbing partition structure, analysis-chip with highly-hydrophobic/water-absorbing complex partition structure, analysis-chip with clear area as partition structure are: the minimization of the height of partition structure enables the maximization of reactor numbers or/and the minimization of sample volume or/and easy washing; and laser confocal scanner or laser scanner can be used directly for scanning.

The advantages of the invented chip with striped-capillary reaction-chamber are: it overcomes not only the disadvantages of the chip with the closed chamber, such as inequable distribution of media (e.g. the appearance of air bubble) and more sample and marker needed, but also the defects of capillary chip with complicated manufacture and high cost, so it has the advantages of simple and easy manufacturing, high sensitivity, less consumed reaction media, convenient performance and etc.

The advantages of the invented chip with convex flow-path are: the flowing speed can be controlled by selecting different material of convex; the preparation is simple and the cost is low.

The advantages of the invented chip with immobilized marker are: with the controlled marker-releasing system, Hook effect can be reduced; therefore the sensitivities of test and device are improved; by simplifying the operation procedures and mechanical transporting system, the efficiencies of test and test device are raised, and automatic test is made available; under the dried condition, marker will have much higher stability.

The advantages of analysis-chip for selective use in this invention are: In one test, only some reactors on the chip will be involved as required, while others can be well preserved, thereby cost is reduced.

### Illustrations

Picture 1A is the schematic diagram of the invented analysis-chip with multiple striped-capillary reaction-chambers;
Picture 1B is the sectional drawing (along a - a line) of the analysis-chip showed in Picture 1A;
Picture 2A -2E are the schematic diagram of different types of reactors and structures of the invented chips;
Picture 3A -3B are the vertical view (downwards) of the bottom unit of the enclosing structure of the invented striped-capillary reaction-chambers;
Picture 3C and 3D are the vertical view (upwards) of the top unit.
   1. Striped-capillary reaction-chamber
   2. Reactor structure
   3. Convex of marking system
   4. Open partition structure
   5. Top plane
   6. Bottom plane
   7. Substrate probe region
   8. Closed partition structure
   9. Inlet
   10. Outlet
   11. Top unit
   12. Bottom unit
   13. Inlet structure on the top plane
   14. Outlet structure on the top plane
   15. Fixing structure on the top plane
   16. Seal structure on the top plane

### Detailed description

### Definition of terms

In this invention, the term "analysis-chip" or "chip" in short, refers to a test device for qualitative and/or quantitative analysis, wherein the result of the specific reaction between a small amount of probe-ligand and the target molecule in a sample can be identified in an addressable way. Analysis-chip includes, but not only includes, the "biochip", "microarray", "bioarray". The analysis-chip includes micro-channeled analysis-chip (e.g. micro-channeled-biochip) and microarray analysis-chip (e.g. biochip, microarray, bioarray etc.), but as known to all it doesn't include the available rapid test strip. The analysis-chip in this invention contains a mono-reactor or multi-reactors with or without the marking system, in which the probe-ligand is immobilized on a substrate with a probe-spot density more than 10 spots/cm² and a probe-spot covering area not larger than 1mm²/spot.

In this invention, the term "test device" in this invention refers to articles used in the quantitative or/and qualitative analysis, which contain probe-ligand reacting with the target in a sample, e.g. instrument and consumable which contain probe-ligand, and kit containing probe-ligand and marking-ligand. Some examples of the test device are: analysis-chip, ELISA plate, affinity electrophoresis strip, affinity chromatography column, planar chromatography reagent strip, analysis-chip kit, ELISA plate kit, affinity electrophoresis kit, etc. The quantitative or/and qualitative analysis can be performed either in vitro, or in vivo.

In this invention, the term "probe-ligand" refers to all active materials that can be immobilized on a solid support in an addressable way so that the target in a sample can be captured by probe-ligand, e.g. DNA, polypeptides, cells, tissues and bio-components etc.

In this invention, the term "ligand" refers to the material that is used to: 1) capture its ligate through interaction (including affinity effect, ion exchange, oleophilic effect, etc.), and 2). bind with labeling material to generate marker. For example, it includes one or more of the following materials: antigens, antibodies, ligates, aptamer screened by Systematic Evolution of Ligands by Exponential Enrichment (SELEX), ligands, polypeptides, polysaccharides, coenzyme, cofactors, antibiotics, steroid, viruses, cells, biotin, avidin etc.

In this invention, the term "labeling material" refers to materials generating or taking part in the formation of the detection signal, such as Rhodamin, Cy3, Cy5 etc, which are commonly used in the analysis-chip detection.

In this invention, the term "substrate" refers to solid support where probe in an analysis-chip are immobilized.

In this invention, the term "reaction room" refers to the place in a reactor where probe-ligand and target react with each other. The reaction room includes the reaction well and reaction-chamber. The reaction well is an open reaction room in which the probe array is open during the reaction. The reaction-chamber is an closed reaction room in which the probe array is closed in its above part during the reaction. The reaction well includes the substrate well and probe. The reaction-chamber includes the substrate chamber and probe. The substrate well and substrate chamber all include substrates and reactor partition structures.

In this invention, the term "reactor" refers to the whole system consisting of the reaction room and all the connected structures therein. A reactor includes the borderline or partition structure, substrate, probe immobilized on the substrate and other connected structures (e.g. flow-path, inlet structure, outlet structure, the fixed marker, etc.). In this invention, the biochip is defined as mono-reactor biochip (N=1) and multi-reactor biochip (N =2 or >2) according to reactor number N presented by the chip. The reactor is defined as a flow reactor and a non-flow reactor depending whether the added liquid media can flow directionally in the reactor during the detection. The biochip characterized by the flow reactor and non-flow reactor is defined as a flow analysis-chip and a non-flow analysis-chip respectively. The reactor is defined as the open reactor and non-open reactor respectively depending whether the probe array in the reactor is open in the whole course of detection. Accordingly the biochip characterized by these reactors is defined as the open analysis-chip or non-open chip, respectively. The reactor of said analysis-chip can simultaneously be featured with several types of reactors mentioned-above. In this invention, this kind of reactor is defined according to all the properties it possesses. The analysis-chip characterized by this type of reactor is defined in the same way. For example: when a probe array is open with no covering from above during the detection, and liquid media added flow directionally in the reactor, this reactor is defined as an open flow reactor; the corresponding analysis-chip is defined as an open flow biochip; others are analogous of this.

In this invention, the term "base-plate" refers to the intermediate used for the preparation of chip, which hasn't been set with the probe. It is based on the substrate and combined with or without other structures (e.g. the partition structure). It will form an analysis-chip after being fixed with the probe-ligand. There may be one or more substrate regions on a base-plate. A single base-plate refers to the one without the partition structure. In such a case, a base-plate is just a substrate, (for example the amino glass slide available in market). A base-plate with partition structure is referred to as the base-plate with more than one of substrate probe regions. In such a case, a base-plate is composed of the substrate and partition structure. Once the probe-ligand is immobilized in a substrate ligand region, a reactor is formed. The base-plate with multiple substrate probe regions thus can form an analysis-chip with multi-reactors.

In this invention, the term "reactor structure" refers to any indispensable structure for a reactor besides the substrate and probe, such as the partition structure, flow-path structure etc.

In this invention, the term "convex" refers to a protruding structure whose height is more than 0 in relation to the plane of the substrate probe region, e.g.

In this invention, the term "reactor partition structure" refers to the structure that coating, adherend etc.can prevent cross-contamination among reactors at least in the course of sample subjectiong. It includes the structures used to segregate part or all of the reactor structures (e.g. reaction well, reaction-chamber, flow-path, inlet structure, outlet structure, etc.). The reactor partition structure includes open and closed partition structures. The open partition structure refers to the reactor partition structure without a cover when the analysis-chip is in use. Conversely, the closed partition structure refers to the reactor partition structure with a cover when the analysis-chip is in use.

In this invention, the term "reactor flow-path" refers to the flow-path network in the reactor. The convex flow-path of the chips of this invention refers to the flow-path higher than the substrate plane, which contains water-absorbing or/and highly-hydrophilic material.

In this invention, the term "reactor separation structure" refers to the structure in the reactor of chip, which presents sample-separation function.

In this invention, the term "marking system" refers to a system in the analysis-chip (including analysis-chip device and analysis-chip kit), which is used for marking the reaction in the reactor of the chip.

In this invention, the term "reactor protecting system" refers to a system that maintains the reactor activity in storage and transporting process, and also keeps the unemployed reactors from being contacted by reaction media when other reactors are in use. The reactor protecting structure is different from the reactor partition structure.

In this invention, the term "polypeptide" includes natural or synthetic proteins, protein fragments, synthetic peptides, etc. Polypeptide includes the common targets in immunoassay and the popular ligands in detection, e.g. antigens, antibodies, etc.

In this invention, the term "nano-particle" refers to solid support particle with a size of less than 500nm, optimally 1-100nm, at least in one-dimension of its three-dimensions.

In this invention, the term "chip with convex flow-path" refers to an analysis-chip comprising convex flow-path; the term "analysis-chip with highly-hydrophobic partition structure" refers to an analysis-chip containing the partition structure with the highly-hydrophobic convex; the term "analysis-chip with water-absorbing partition structure" refers to an analysis-chip containing the partition structure with the water-absorbing convex; the term "analysis-chip with blank region partition structure" refers to an analysis-chip containing the partition structure with substrate blank region of width is 0.5-10mm,and optionally, hydrophobic convex; the term "analysis-chip with highly-hydrophobic/ water-absorbing partition structure" refers to an analysis-chip containing the partition structure with the highly-hydrophobic convex and the water-absorbing convex; the term "analysis-chip with fixed marker" refers to an analysis-chip containing fixed marker; the term "optionally-using analysis-chip" in the invention refers to an analysis-chip with multi-reactors, where only part of reactors are to be involved in the testing if needed, without interfering with other reactors

Term "analysis-chip with highly-hydrophobic partition structure" in the invention refers to the chip containing the partition structure with highly-hydrophobic convex.

Term "analysis-chip with water-absorbing partition structure" in the invention refers to the chip containing the partition structure with water-absorbing convex.

Term "analysis-chip with clear area as partition structure" in the invention refers to the chip containing the partition structure including clear area on substrate with a width of 0.5-10mm and optionally hydrophobic convex.

Term "analysis-chip with highly-hydrophobic or/and water-absorbing complex partition structure" in the invention refers to the chip containing the partition structure with highly-hydrophobic or/and water-absorbing complex convex.

Term "analysis-chip with immobilized marker" in the invention refers to the chip containing immobilized marker.

Term "analysis-chip for selective use" in the invention refers to the chip with multi-reactors, where only part of reactors can be involved in the testing if needed, without interfering with other reactors.

In this invention, the term "water contact angle" refers to the water contact angle on a surface. For a long time, it has been widely acknowledged that contact angle θ of liquid drop on the surface of a solid substance can be taken as the quantitative indicator of some specific moisture capacity. If liquid is dispersed entirely on the surface and forms a membrane, then the contact angle θ is zero. If certain angle is formed between the drop of liquid and the surface of substance, the surface is not considered wet. Glass is the most commonly used material as the substrate of solid support of chips, whose water contact angle is about 45°

With the following implementation examples, more detailed explanations of the invention will be given. However, it should be pointed out that these examples are only the individual cases concerning the implementation of this invention. Professionals should learn more about other aspects of the implementation based on these examples.

The substrate in the implementation example of this invention includes hydrophilic substrate and hydrophobic substrate. The former includes the epoxy glass slide and epoxy-group cover glass made by epoxidation. The latter is the glass slide painted black made by ourselves. The glass slides are purchased from USA ESCO SCIENTIFIC Comp. The dimension is 76×26×1.0mm; the cover glass is 60×24×0.15mm. The probes purchased from Hepatic Disease Institute, Beijing People Hospital are HIVI+2 antigens, HBs antigens, HCV antigens, HBs antibodies respectively. The spotting concentration is within 1.0-1.5mg/ml. In the implementation examples, Sample 1 is HCV antibody-positive serum. Sample 2 is HIV1+2 antibody-positive human serum. Sample 3 is HBs antibody-positive human serum. Sample 4 is HBs antigen-positive human serum. Sample 5 is a negative control. All the samples are predetermined under the same testing condition by the classic open analysis-chip with the mono-reactor.

### Implementation example 1: Preparation of the analysis-chip with the highly-hydrophobic partition structure as well as the application thereof

In this implementation example, the hydrophobic liquid in use is "polyacrylate paint" (made by China Chengdu Chenguang Research Institute of Chemical Industry, with a water contact angle as 85°), "organosilicon water-proof paint" (made by China Chengdu Chenguang Research Institute of Chemical Industry, with a water contact angle as 116 ° ), "highly-hydrophobic latex paint"(supplied by China Chengdu Chenguang Research Institute of Chemical Industry, with a water contact angle as 123°),"highly-hydrophobic silica paint" (supplied by China Zhoushan Mingri Nanomaterials Co., Ltd., with a water contact angle as 151°) respectively. The highly-hydrophobic solid substances in use herein are "polytetrafluoroethylene pressure-sensitive adhesive tape" (supplied by China Chengdu Chenguang Research Institute of Chemical Industry, with a water contact angle as 117°), and "nanometer textile" (supplied by China Zhoushan Mingri Nanomaterials Co., Ltd., with a water contact angle as 155°) respectively. Although the partition structure of the invented reactor could partially or entirely be the device of this invention, this implementation example only takes the simplest cases. Other cases may be illustrated in the following implementation examples.

### 1) The preparation of the substrate with highly-hydrophobic partition structure

### (1) Preparation of the substrate with highly-hydrophobic partition structure by solidifying the highly-hydrophobic liquid material

Coat said highly-hydrophobic liquid materials onto the borderline of the reactor on the epoxy-group slide. In accordance with the supplier's instruction, solidify it after it is dried at room temperature, form a highly-hydrophobic convex whose height is 25-115um and whose width is 2.0-2.5mm, and make the convex into various geometric shapes. In this example, only two types are used: strip (the convex is in a shape of strip) and line combination (e.g. the convex has two lines with space in between). The surface enclosed by highly-hydrophobic convex could be in various geometric figures. In this example, 3mm × 3mm rectangle is used. On the surface of the substrate, there are 20 substrate-wells altogether, 10 substrate-wells being arranged lengthwise and 2 substrate-wells transversely.

### (2) Preparation of the substrate with highly-hydrophobic partition structure by immobilizing the highly-hydrophobic solid materials

Adhere the highly-hydrophobic solid material onto the borderline of the reactor on the epoxy glass slide to form a highly-hydrophobic convex whose height is 105-435um, and whose width is 2.0-2.5mm. The surface enclosed by the highly-hydrophobic convex could be in various geometric shapes. In this example, the surface enclosed by highly-hydrophobic convex is a 3mm × 3mm rectangle. On the surface with highly-hydrophobic convex of the substrate, there are 56 substrate-wells altogether, 14 substrate-wells being arranged lengthwise, and 4 substrate-wells transversely.

### 2) The preparation of the analysis-chip with the highly-hydrophobic partition structure

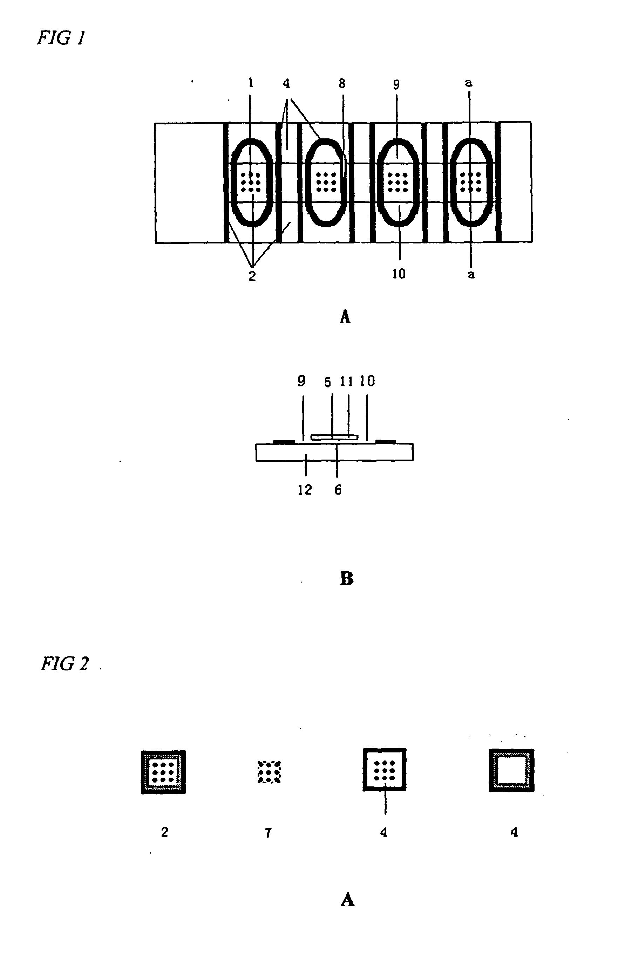

In fact, the invented analysis-chip can be fixed with probes first and then go on with the preparation of the highly-hydrophobic convex; or the other way around: preparing the highly-hydrophobic convex first and then fixing the probe later. This example adopts the second method. In the probe region 0.5mm away from said highly-hydrophobic convex in the substrate-well, 3 types of antigens mentioned-above are immobilized by the known method of probe spotting. A 3 × 3 probe array thus comes into being, with each antigen for 3 spots. Then block the analysis-chip with bovine serum albumin. The final structure of reactor (Fig 2A) includes the probe region on substrate and open partition structure.

### 3) Evaluation and applications of the analysis-chip with the highly-hydrophobic partition structure

In the experiment, take 10 chips of each type mentioned-above, number the reaction wells, any of the two reaction wells border upon are added respectively the positive serum of the mixture of the same volumes of Sample 1, 2, 3 and the negative control serum. The marker used is the mouse monoclonal antibody labeled with Rhodamine, which is self-made with the known method. In the experiment, the sample subjecting volume is 10µl; the marker subjecting volume is 10µl. When the sample is added and after the reaction is completed, there is no need to suck all the unbound substance in the reaction wells before washing as performed in ELISA. Instead, washing is conducted in accordance with one of the following approaches: (a) suck the unbound substances in the reaction well slightly with absorbent paper. And then rinse it for 1 minute with wash solution; (b) rotate the slide to form an angle of 45° with the horizontal plane, and then rinse it for 1 minute by spraying washing solution downward; (c) rotate the slide to form an angle of 180° with the horizontal plane, and then rinse it for 1 minute by spraying washing solution upward. The marker is added, washed, and dried with the known methods. After being dried, it is scanned with scanners. The scanner used herein is a laser confocal scanner (Afymetrix, GMS 418 microarray scanner), with wavelengths of excitation light at 532nm; emission light at 570nm. The visualized signal is processed by software (JAGUAR II). The cross-contamination rate is defined as follows: the numbers of reaction wells, in which the acquired results disagree with the added samples, is divided by total numbers of reaction wells examined. The experimental results are showed in Table 2:

**Table 2**

| Chip | Highly-hydrophobic material | Substrate | The thickness of convex | Convex structure | Blank space | Cross-Contamination rate |
|---|---|---|---|---|---|---|
| 1 | Polyacrylate paint | Epoxy slide | 75-105µm | Strip | >500µm | 0 |
| 2 | Organosilicon water-proof paint | Epoxy slide | 20-115µm | Strip | >500µm | 0 |
| 3 | Organosilicon water-proof paint | Epoxy slide | 20-89µm | Strip | >500µm | 0 |
| 4 | Organosilicon water-proof paint | Epoxy slide | 15-106µm | The line combination of | >500µm | 0 |
| 5 | Highly-highly-hydrophobic latex paint | Epoxy slide | 75-105µm | Strip | >500µm | 0 |
| 6 | Highly-hydrophobic silica paint | Epoxy slide | 65-89µm | Strip | >500µm | 0 |
| 7 | Polytetrafluoroethylene pressure-sensitive adhesive tape | Epoxy slide | 120µm | Strip | >500µm | 0 |
| 8 | Nanometer textile | Epoxy slide | 400µm | Strip | >500µm | 0 |
| 9 | Nanometer textile | Black paint slide | 400µm | Strip | >500µm | 0 |

### Implementation example 2: Preparation of the analysis-chip with the water-absorbing partition structure and applications thereof

In this example, the water-absorbing materials in use are: "starch grafting acrylic acid water-absorbing membrane (whose thickness is 80-100µm, water absorbability 450g/g, provided by Chenguang Research Institute of Chemical Industry)", "water-absorbing paper (whose thickness is 80-100µm, water absorbability 150g/g, provided by Chenguang Research Institute of Chemical Industry)" and deacetylated chitin grafting acrylonitrile water-absorbing membrane (whose thickness is 80-100µm*,* water absorbability 220g/g, provided by Chenguang Research Institute of Chemical Industry)" respectively. Although the partition structure of the invented reactor could be partially or entirely the device of this invention, this example only takes the simplest cases.

### 1) Preparation of the substrate with water-absorbing partition structure

First, punch 3mm holes in said water-absorbing solid, adhere the material onto the epoxy glass slide, and then form a water-absorbing convex with 3mm upright holes whose height is 105-435um, and the space between the two holes is 2.0-2.5mm. The convex can be in various geometric figures. In this example, only strip figure is used (convex is in a shape of a strip). On the surface of the substrate, there are 20 wells (substrate-well) altogether, 10 wells in lengthwise direction and 2 wells in transverse direction.

### 2) Preparation of the analysis-chip with water-absorbing partition structure

The preparation method is the same as that of analysis-chip with highly-hydrophobic partition structure in Implementation example 1. The analysis-chip is blocked with bovine serum albumin, dried and is made ready for use.

### 3) Evaluation and applications of the analysis-chip with water-absorbing partition structure

In the experiment, take 10 chips of each type mentioned-above, number the reaction wells, any of the two reaction wells border upon are added respectively the positive serum of the mixture of the same volumes of Sample 1, 2, 3 and the negative control serum. The subjecting volume of sample is 7µl; that of marker is 7µl. The experimental method is the same as that in Implementation example 1. The cross-contamination rate is defined as follows: the numbers of reaction wells, in which the acquired results disagree with the added samples, is divided by total numbers of reaction wells examined. The cross-contamination rate on the analysis-chip prepared in this example is zero.

### Implementation example 3: Preparation of analysis-chip with a blank region as partition structure and the applications thereof

In this example, the hydrophobic materials in use are: "black polyacrylate paint" (supplied by China Chengdu Chenguang Research Institute of Chemical Industry, with a water contact angle as 78°), "black paint" (supplied by China Chengdu Chenguang Research Institute of Chemical Industry, with a water contact angle as 76°). The substrate used herein is epoxy glass slide. Although the partition structure of the invented reactor could be partially or entirely the device of this invention, this example only takes the simplest cases.

### 1) Preparation of the substrate containing the marking line with a blank region as partition structure

Coat the hydrophobic paint mentioned-above on the epoxy glass slide to form a black marking line whose height is 30-40µm, and whose width is 500-1000µm. On the surface of the substrate, there are 56 substrate-wells altogether, 14 in lengthwise direction, and 4 in transverse direction.

### 2) The preparation of the analysis-chip with a blank region as partition structure

### (1) The preparation of analysis-chip with a completely blank region as partition structure

The partition structure with a completely blank region doesn't contain hydrophobic convex. It is made by directly immobilizing several probe arrays onto the surface of the substrate. The immobilization of each probe array in this example is the same as that in Implementation example 1. There are 10 probe arrays immobilized lengthwise and 2 probe arrays immobilized transversely, 20 probe arrays altogether. The substrate blank region between every two probe-arrays is 4.5mm in width. The analysis-chip is blocked with bovine serum albumin and made ready for use.

### (2) Preparation of the analysis-chip containing the marking line with a blank region as partition structure

Said preparation is based on the substrate prepared above, which contains the marking line with a blank region as partition structure. The preparing method is the same as that in Implementation example 1.

### 3) Evaluation and applications of the analysis-chip with a blank region as partition structure

In the experiment, take 10 chips of each type mentioned-above, number the reaction wells, any of the two reaction wells border upon are added respectively the positive serum of the mixture of the same volumes of Sample 1, 2, 3 and the negative control serum. The subjecting volume of sample is 7µl; that of marker is 7µl. The experimental method is the same as that in Implementation example 1. The cross-contamination rate is defined as follows: the numbers of reaction wells, in which the acquired results disagree with the added samples, is divided by total numbers of reaction wells examined. The cross-contamination rate on the analysis-chip prepared in this example is zero.

### Implementation example 4: Preparation of the analysis-chip with highly-hydrophobic/water-absorbing complex partition structure and the applications thereof

In this example, the highly-hydrophobic liquid substance in use is "organosilicon water-proof paint" (supplied by China Chengdu Chenguang Research Institute of Chemical Industry, with a water contact angle as 116°); the water-absorbing material used herein is "starch grafting acrylic acid water-absorbing membrane (whose thickness is 80-100µm, water absorbability 450g/g, provided by Chenguang Research Institute of Chemical Industry)". Though the partition structure of invented reactor could be made up partially or entirely of the device of the invention, this example only presents the simplest cases as the samples.

### 1) Preparation of the substrate with water-absorbing partition structure

First, punch 3mm holes in said water-absorbing solid, painting the said highly-hydrophobic paint with 0.5-1.0mm width onto the said water-absorbing solid near the each hole, adhere the material onto the borderline of the reactors on the epoxy glass slide, and then form a water-absorbing convex with 3mm upright holes whose height is 105-435µm, and the space between the two holes is 2.0-2.5mm. The convex can be in various geometric figures. In this example, only strip figure is used (convex is in a shape of a strip). On the surface of the substrate, there are 20 wells (substrate-well) altogether, 10 wells in lengthwise direction, and 2 wells in transverse direction.

### 2) Preparation of the analysis-chip with the highly-hydrophobic/water-absorbing partition structure

The preparing method is the same as that of the analysis-chip with highly-hydrophobic partition structure in Implementation example 1. The analysis-chip is blocked with bovine serum albumin, dried and then is made ready for use.

### 3) Evaluation and applications of the analysis-chip with the highly-hydrophobic/water-absorbing partition structure

In the experiment, take 10 chips of each type mentioned-above, number the reaction wells, any of the two reaction wells border upon are added respectively the positive serum of the mixture of the same volumes of sample 1, 2, 3 and the negative control serum. The subjecting volume of sample is 7µl; that of marker is 7µl. The experimental method is the same as that in Implementation example 1. The cross-contamination rate is defined as follows: the numbers of reaction wells, in which the acquired results disagree with the added samples, is divided by total numbers of reaction wells examined. The cross-contamination rate on the analysis-chip prepared in this example is zero.

### Implementation example 5: Preparation of the analysis-chip with the controlled marker releasing system and the applications thereof

The invented analysis-chip with the controlled marker releasing system is actually an analysis-chip with a marker release-controlling system in the reactors. Similar to the drug release-controlling system, it also includes a programmable release system and an intelligent release system. The latter includes a release system with the external adjustment and intelligent gel. The marker release-controlling system of this invention can be prepared the same way as the existing drug release-controlling system.

For the programmable drug release system, the methods of dissolving system can be prepared via using the hydrogel microsphere of ortho-hydroxybenzoic acid cross-linked by Ca²⁺ ion to embed drug, using the hollow body of polylactic acid to embed drug, using the hollow body of polylactic acid to embed foaming agent, using microsphere of water soluble polymers such as hydroxy-propyl cellulose or hydroxy-propyl methyl cellulose to embed drug, or using the intelligent drug release system with the external adjustments (e.g. through light, heat, PH value, electromagnetic, ultrasonic wave etc.).

Though this example only offers the simplest cases for the preparation of the invented controlled marker releasing system, it is clear to professionals that the method mentioned here can be used to prepare a variety of controlled marker releasing systems.

### 1) The half-release period of the markers with controlled release system switched on by solvent

In this example, the controlled marker releasing system is composed of the controlled-releasing agent and membrane-enclosed powder marker formed by excipient. The marker used here is enzyme-labeled goat-anti-human secondary antibody (Beijing Tiantan Biological Products Co., Ltd.). The controlled-releasing agent used herein is the water-soluble organics (see Table 3) whose coating thickness is 80-100µm. The controlled release system is switched on by dissolution with PBS buffer solution as a solvent. First, the solution of controlled-releasing agent with proper concentration will be coated onto the slide, forming a coating of 1 mm × 1 mm × 0.1mm (length × width × height). After being dried, this coating is again painted with concentrated marker solution. After the marker is dried, the concentrated solution of controlled-releasing agent is coated to enclose the marker and is dried again for future use. At 37°C, a certain amount of solvent will be added to the controlled release system. The soluble parts will be taken out in different time. After that, half-release period can be calculated by detecting the dissolved marker through antibody-secondary antibody reaction. The half-release periods of marker in some controlled release systems switched on by solvent are showed in Table 3.

**Table3**

| Controlled-releasing agent | Half-release period (minute) |
|---|---|
| Starch | 2 |
| Gelatin | 3 |
| Hydroxypropyl methyl cellulose | 5 |
| Hydroxypropyl cellulose | 12 |
| Acacia | 15 |

### 2) Preparation of the analysis-chip with the controlled marker releasing system

The marker in the marking convex on said analysis-chip of this example is Rhodamine-labeled goat-anti-human secondary antibody. Acacia and ethyl cellulose are used as controlled-releasing agents. The substrate used herein is the substrate of analysis-chip with highly-hydrophobic partition structure based on epoxy glass slide prepared in Implementation Example 1.

### (1) Fixing the marking convex in the periphery of the probe array in the reactor

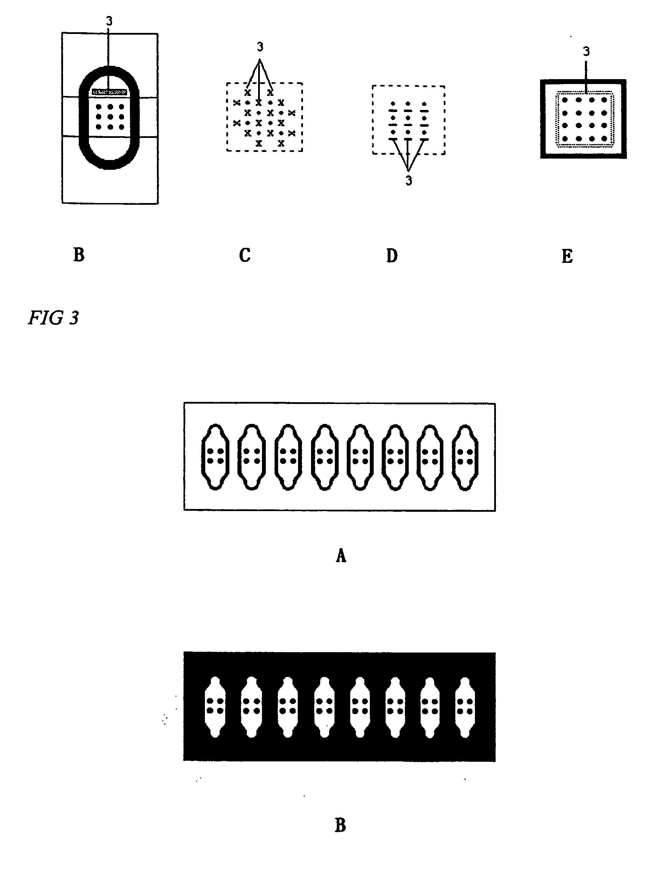

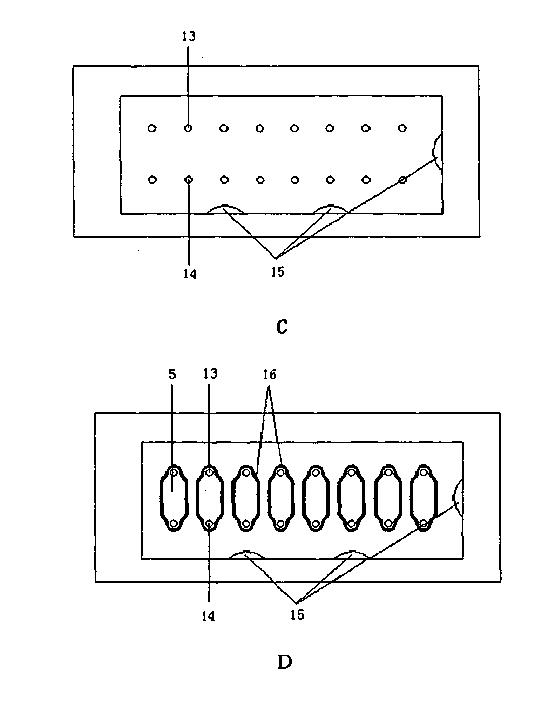

In implementation example 1, said three antigen probes are immobilized in the substrate well to form a probe array, which is then blocked with bovine serum albumin solution. Then Acacia with proper concentration is painted around bottom of the reaction well, forming a 1 mm-wide strip. After being dried, the marker containing starch as excipient is coated onto the strip of Acacia, which is dried again and coated with Acacia for enclosing. After it is dried, it is made ready for use. The marking convex prepared in this way has a thickness of 350-480µm. Thereby in the prepared reactor structure (Fig. 2E), the marking convex 3 is outside the substrate probe region with the open partition structure around it. Of course, the marking convex can also be surrounded by the closed partition structure (Fig. 2B). And the half-release period of the marker at 37°C is tested for 15 minutes.

### (2) Fixing the marking convex inside the probe region to form the probe/marking system arrays

Following the probe-spotting method in implementation example 1, the marker solution containing gelatin (secondary antibody 1mg/ml) at a proper concentration and probe solutions of three antigens are spotted respectively into the substrate-well of the substrate to form the probe/marker arrays. The reactor (Fig. 2C, 2D) thus obtained has the array of probe/marking convex 3 inside the substrate probe region, surrounded with the open partition structure. The half-release period of the controlled release system at 37°C is tested for about 3 minutes.

### 3) The application of the analysis-chip with the controlled marker releasing system

In experiment, four samples are subjected into said analysis-chip with the controlled marker releasing system respectively, with each sample in 2 reaction wells. The sample subjected should be diluted properly. In this example, 5µl of samples should be subjected and kept under incubation at 37°C for 5 minutes. After that, washing should be conducted for 3 times, with 15µl of washing solution added for each washing. And then, after 15µl of diluent is subjected, there is another 5-minute incubation at 37°C. After reaction, it is rinsed 5 times with 15µl of washing solution added each time. Scanning (with GMS 418 microarray scanner made by Afymetrix,) will be conducted after drying. The processed data are listed in Table 4.

**Table 4**

| Method | | Standard ELISA method | Controlled marker releasing method |
|---|---|---|---|
| HCV antibody-positive serum | HCV antibody | + | + |
| | HIV antibody | - | - |
| HIV antibody-positive serum | HCV antibody | - | - |
| | HIV antibody | + | + |
| Positive control | HCV antibody | + | + |
| | HIV antibody | + | + |
| Negative control | HCV antibody | - | - |
| | HIV antibody | - | - |

| | | | |
|---|---|---|---|
| "+" stands for the positive result; "-" the negative result. | | | |

### Implementation example 6: Preparation of the analysis-chip with the convex flow-path and the applications thereof

This implementation example illustrates how a micro-channel path is prepared with highly-hydrophilic material. The invented analysis-chip with highly-hydrophilic micro-channel path differs from the present micro-channel path analysis-chip mainly in that the former employs the surface of highly-hydrophilic material as an important component for micro-channel path.

### 1) Preparation of the highly-hydrophilic micro-channel path without the partition structure

Though there are many highly-hydrophilic materials with varied hydrophilicities available for selection, this implementation example illustrates only two materials. The highly-hydrophilic liquid material used herein is "highly-hydrophilic nanometer silica paint" (provided by China Zhoushan Mingri Nanomaterials Co., Ltd., with a water contact angle as 28°) and "highly-hydrophilic polyacrylic acid paint" (supplied by China Chengdu Chenguang Research Institute of Chemical Industry, with a water contact angle as 35°).

The highly-hydrophilic liquid material is used to draw a line with a width of 80-100µm and with a length of 10mm on the surface of the slide. After being dried, it is ready for use.

### 2) Preparation of the highly-hydrophilic micro-channel path with the partition structure

"Organosilicon water-proof paint" (supplied by China Chengdu Chenguang Research Institute of Chemical Industry whose water contact angleis 116°) is coated onto both sides of said highly-hydrophilic micro-channel path without the partition structure, with a width of 1mm. After being dried, the highly-hydrophilic micro-channel path with the partition structure is ready

### 3) Applications of the highly-hydrophilic micro-channel path

Set said slide horizontally. Subject 10µl of PBS buffer solution into one end of the prepared highly-hydrophilic micro-channel path. As a result, a phenomenon similar to capillarity occurs instantly, that is, the liquid gradually flows from the liquid-subjecting end to the other end.

It is easy for professionals to apply the invented micro-channel path to the analysis-chip with micro-channel path, such as PCR chip, micro-extraction chip, chemical synthetic chip, cell counting chip, integrated analysis-chip with multi-system, bio-microarray, multi-channeled integrated chip, capillary electrophoresis chip, lab-on-chip, etc.

### Implementation example 7: preparation of optionally-using analysis-chip and the applications thereof

The analysis-chip prepared in this example is an analysis-chip in which the number of reactors to be used can be selected according to the analysis, includes two types: the analysis-chip in which selected reactors can be detached, and the analysis-chip in which selected reactors can be opened, according to the need of the analysis. In the former, substrate can be easily dismantled; while in the latter, reactor is covered by sealing unit.

### 1) Preparation of the detachable chip

The easily-detachable substrate used in this implementation example is a polystyrene plate with a precut ditch that can help dismantle the substrate if needed. The area surrounded by the precut ditch is 4 × 4 mm. The ditch is 1mm wide and 0.8mm deep. Then "Organosilicon water-proof paint" (supplied by China Chengdu Chenguang Research Institute of Chemical Industry, with a water contact angleas 116°) is painted onto the border of the area surrounded by the precut ditch, forming a highly-hydrophobic convex line with a height of 80-100µm and a width of about 1mm. Thereby a substrate-well comes into being. After that, said HCV fusion antigen, HIV1+2 fusion antigen, and HBs antigen are spotted respectively in 3 spots into each substrate-well, forming a 3 × 3 arrays; while the areas on the substrate without being spotted will be blocked and dried for future use. The same preparation method is applicable to other detachable chips with an easily-detachable substrate (e.g. the activated glass slide, metal plates, plastic plates etc., which have undergone the pre-cutting).

### 2) Preparation of the openable chip

The reactor-sealing unit used in this implementation example includes: the plastic plate with a removable area made by mechanic cutting, aluminous plastic film, and plastic film with or without removable parts, etc.

Spotting is conducted in the same way as mentioned above on the substrate prepared in Implementation example 3, which contains a marking line, with a blank region as the partition structure. After 20 reaction wells are formed on the substrate, waterproof pressure-sensitive adhesive with a height of 80-100µm, and a width of about 1mm is coated onto the border of the reactor well, which will then be enclosed by reactor-sealing units.

### 3) Application of the optionally-using analysis-chip

In testing, different number of reaction wells can be taken as required by dismantling parts of the substrate in the detachable analysis-chip along the precut ditch or by uncovering the easily-removable part of said openable analysis-chip with vacuum pumps, or removing the covers above the reaction well by mechanic cutting while keeping other reaction wells intact. In the experiment, take 10 chips of each type mentioned-above, number the reaction wells, any of the two reaction wells border upon are added respectively the positive serum of the mixture of the same volumes of sample 1, 2, 3 and the negative control serum. The subjecting volume of sample is 7µl; that of marker is 7µl. The experimental method is the same as that in Implementation example 1. The cross-contamination rate is defined as follows: the numbers of reaction wells, in which the acquired results disagree with the added samples, is divided by total numbers of reaction wells examined. The cross-contamination rate on the analysis-chip prepared in this example is zero.

### Implementation example 8: Application of the analysis-chip with the high-density reactor and the applications thereof

### 1) Preparation of the analysis-chip with the high-density reactor

In the example, the prepared analysis-chip with the high-density reactor uses epoxy glass slide as the substrate. Hydrophobic liquid material "polyacrylate paint" (supplied by China Chengdu Chenguang Research Institute of Chemical Industry, with a water contact angle as 85°) is coated onto the substrate to form the highly-hydrophobic convex with a height about 100µm, and a width about 1mm, and form a substrate-well of 2 × 2mm. After that, 3 types of antigen solutions will be deposited with an arrayer (GM417ARRAYER, GENETIC MICROSYSTEM COMP.) into said reserved area in the pattern of 10 spots for each type of ligand, so as to form 5 × 6 probe arrays. The probe array (the substrate probe region) is 1 × 1 mm in size. The density of reaction well on the substrate is over 9 units/cm² .

### 2) Evaluation and application of the analysis-chip with the high-density reactor

In the experiment, take 10 chips mentioned-above, number the reaction wells, any of the two reaction wells border upon are added respectively the positive serum of the mixture of the same volumes of Sample 1, 2, 3 and the negative control serum. The subjecting volume of sample is 5µl; that of marker is 7µl. The experimental method is the same as that in Implementation example 1. The cross-contamination rate is defined as follows: the numbers of reaction wells, in which the acquired results disagree with the added samples, is divided by total numbers of reaction wells examined. The cross-contamination rate on the analysis-chip prepared in this example is zero.

### Implementation example 9: Preparation of analysis-chip with the striped-capillary reaction-chamber and the application thereof

### 1) Preparation of the monoplane analysis-chip with the striped-capillary reaction-chamber

The monoplane analysis-chip with the striped-capillary reaction-chamber without the protection unit prepared in the example is marked as A1 (as showed in Picture 1). The monoplane analysis-chip with the striped-capillary reaction-chamber containing the protection unit is marked as A2.

The unit containing probes is prepared as below: on this substrate, "organosilicon highly-hydrophobic paint" (Chengdu Chenguang Research Institute of Chemical Industry) is coated to form a partition strip on the scheduled 8 inlet and outlet areas as shown in Picture 1. The width of each substrate-well is 4mm. After it is dried overnight, deposit above HCV fusion antigen solution and HIV fusion antigen solution in the pattern of 3 spots per each ligand into the above scheduled areas with an arrayer (GM417ARRAYER, GENETIC MICROSYSTEM COMP), forming 3 × 3 probe arrays. After 3 hours of coating reaction at 37°C, it is blocked with bovine serum, rinsed and dried for use.

The formation of the striped-capillary reaction-chamber: the cover glass (60 X 12.5 X 0.15mm in dimension) is used as the top unit 11; the unit with probes prepared as above is used as the bottom unit 12. The top unit 11 is connected to the bottom unit 12 through adhesion. The used adhesive is dual epoxy resin (omnipotent adhesive, Chengdu Chenguang Research Institute of Chemical Industry). In accordance with the instruction of product, spread the adhesive onto the highly-hydrophobic coating of the unit containing substrates. And then adhere it to the other unit. Of the prepared analysis-chip with the striped-capillary reaction-chamber, the width of its striped-capillary reaction-chamber 1 is 4000µm. The space between the top plane 5 and bottom plane 6 is 80µm. There is a closed partition structure 8 in the parts of the top plane and bottom plane among the striped-capillary reaction-chambers. There are the open partition structure 4, inlet 9, outlet 10 in the parts of liquid inlet and outlet. The water contact angle of the glass slide is 44° .

Preparation of the monoplane analysis-chip with the striped-capillary reaction-chamber containing the protection structure: the inlet and outlet area mentioned-above can be closed or opened according to the need by using the preparation method for the dismantled analysis-chip in Implementation example 7.

### 2) Preparation of the double-plane analysis-chip with the striped-capillary reaction-chamber

The double-plane analysis-chip with the striped-capillary reaction-chamber without the protection unit prepared in this example is marked as A3 (as showed in Picture 1). The double-plane analysis-chip with the striped-capillary reaction-chamber containing the protection unit is marked as A4.

The top unit is based on the amino-group cover glass. The bottom unit is based on the amino-group slide. Both top plane and bottom plane are immobilized with probes. By using the ligand solution mentioned-above, HCV and HIV antigens are deposited respectively in the same pattern onto the corresponding positions of the top and bottom units with an arrayer (GM417ARRAYER, GENETIC MICROSYSTEM COMP.), 2 spots each, forming 2X2 ligand arrays. After the coating reaction, it is sealed, rinsed and dried. The connection of the top and bottom units as well as the formation of the inlet and outlet are the same as in Implementation example 1). Of the prepared analysis-chip with the striped-capillary reaction-chamber, the dimensions of the top plane and bottom plane of the striped-capillary reaction-chamber is 4mm × 12.5mem (width × height). The space between the top plane and bottom plane is 0.08mm. The water contact angle of the glass slide is 44°.

### 3) Examination of characteristics of the analysis-chip with the striped-capillary reaction-chamber

PBS is used as the test media in the implementation example. Set the analysis-chip obtained in 1) and 2) upright (inlet on the bottom, and outlet on the top). Deposit 5ul of PBS with an 8-tipped pipette into the entrance of the striped-capillary reaction-chamber in the inlet well, and observe PBS solution filling the striped-capillary reaction-chamber through the inlet into outlet.

### 4) Utilization of the analysis-chip with the striped-capillary reaction-chamber Sample 1, 2, and 5 are used in the implementation example. Before the samples are subjected, they are diluted properly. There are two approaches for subjecting sample:

(1) Subjecting sample by batches: the volume subject is 3ul. During the processing, when the sample is subjected into the feeding well, the sample will fill the whole striped-capillary reaction-chamber from the inlet to outlet automatically due to capillarity. There isn't air bubbles observed. Put the analysis-chip into an incubator for reaction at 37°C for 5 minutes.
(2) Subjecting sample continually: during the processing, the sample is warmed up to 37 °C. Subject the sample into the feeding well with the flow velocity of 1ul/min. The subject volume is 10ul. The subject time is 10 minutes. The sample will fill the whole striped-capillary reaction-chamber from the inlet to outlet owing to the capillarity. There isn't air bubbles observed. The reaction temperature is 37°C. The reaction time is 5 minutes.

After the reaction, the sample is absorbed with paper or drawn out by the pipette via the outlet. Washing solution could be subjected by batches or continually with the total volume of 20ul.

The marker is Rhodamine-labeled goat anti-human secondary antibody (Jackson Immunoresearch Laboratories). In order to use the marker as few as possible, the batch-subjecting approach is applied. The subject volume is 3µl, the reaction temperature is 37 °C and the reaction time is 5 minutes. Washing after the labeling reaction is the same as that for the sample reaction. After being dried, the results are visualized directly by using the laser confocal microscope scanner (Afymetric Comp, GMS 418 analysis-chip Scanner). The data are processed to get results showed in Table 6.

**Table 6**

| Chip | HCV antibody positive serum | | HIV antibody positive serum | | Positive control | | Negative control | |
|---|---|---|---|---|---|---|---|---|
| | HCV antibody | HIV antibody | HCV antibody | HIV antibody | HCVantibody | HIV antibody | HCV antibody | HIV antibody |
| Subject sample by batches | | | | | | | | |
| A1* | + | - | - | + | + | + | - | - |
| A2* | + | - | - | + | + | + | - | - |
| A3* | + | - | - | + | + | + | - | - |
| A4* | + | - | - | + | + | + | - | - |
| A4** | + | - | - | + | + | + | - | - |
| Subject sample continually | | | | | | | | |
| A1* | + | - | - | + | + | + | - | - |
| A2* | + | - | - | + | + | + | - | - |
| A3* | + | - | - | + | + | + | - | - |
| A4* | + | - | - | + | + | + | - | - |
| A4** | + | - | - | + | + | + | - | - |

| | | | | | | | | |
|---|---|---|---|---|---|---|---|---|
| "+" stands for the positive result; "-" is the negative result "* "and "**" are the results of samples of 10-fold-diluted and of 20-fold-diluted respectively. | | | | | | | | |

### Implementation example 10: Application of the analysis-chip with the irreversible closed striped-capillary reaction-chamber and the application thereof

The substrate (amino-group slide) and two probes (HCV antigen and HIV antigen) in the implementation example are the same as those in Implementation example 1.

### 1) Preparation of the bottom unit -

### (1) The material of the wall and seal structure of the striped-capillary reaction-chamber of bottom unit are elastic material

On the amino-group slide, elastic material (air-drying silicone rubber solution, Chengdu Chenguang Research Institute of Chemical Industry) is used to coat the whole areas evenly except 8 areas (refer to Picture 3B; each area has a width of 4mm) that are scheduled for fixing ligands. The thickness of elastic layer is less than 0.06mm. After it is dried overnight, deposit the above ligands solution, in the pattern of 3 spots per ligand, into the above-scheduled areas with an arrayer (GM417ARRAYER, GENETIC MICROSYSTEM COMP.), forming 3 x 3 probe arrays. After 3-hour coating reaction at room temperature, it is blocked with bovine serum, cleaned and dried for use. The obtained bottom unit is marked as A1.

### (2) The use of highly-hydrophobic material for the bottom unit of the wall and enclosing structure of the striped-capillary reaction-chamber

In the implementation example, the preparation of bottom unit is same as the unit-containing substrate in item 1) of Implementation example 1. The thickness of highly-hydrophobic material layer is less than 0.06mm. The obtained bottom units that use amino glass slide and black paint glass slide are marked as A2 and A3 respectively.

### (3) The use of highly-hydrophobic and elastic materials respectively for the bottom unit of the wall and seal structure of the striped-capillary reaction-chamber

On an amino-group slide, the highly-hydrophobic material (organosilicon highly-hydrophobic paint, Chengdu Chenguang Research Institute of Chemical Industry) is coated onto the border of the 8 areas (as showed in Picture 3A, each area has a width of 4mm) scheduled for fixing ligand to generate a borderline layer. After it is dried, elastic material (air-drying silicone rubber solution, Chengdu Chenguang Research Institute of Chemical Industry) is coated onto the areas outside the highly-hydrophobic borders, forming a strip whose width is 2mm. The layer of the elastic coating is a little higher than the highly-hydrophobic layer (The thickness of layer is less than 0.05mm). And then it is coated with the ligand solution mentioned-above. The coating method is the same as (1) in 1) of this implementation example. The obtained bottom unit is marked as A4.

### (4) The material of the wall and the enclosing structure of striped-capillary reaction-chamber of bottom unit is adhesive material

On amino-group slide, adhesive material (water-insoluble adhesive tape using polytetrafluoroethylene as substrate, Chengdu ChenGuang Research Institute of Chemical Industry) is used to adhere the areas outside 8 areas scheduled for fixing ligands (as showed in Picture 3B. each area with a width of 4mm) homogenously (the thickness of layer is less than 0.2mm). And then it is spotted with ligand solutions mentioned-above. The spotted method is the same as (1) in 1) of the implementation example. The obtained bottom unit is marked as A5.

### (5) The bottom unit containing the striped-capillary reaction-chamber without the seal structure

The ligands mentioned-above are coated onto the amino glass slide directly. The coating method is the same as (1) in 1) of the implementation example. The obtained bottom unit is marked as A6.

### 2) Preparation of the top unit

(1) Top unit without the formation structure
   The top unit (See Picture 3C) is made from stainless steel plate which can be used repeatedly, with a size as 100mm × 40mm × 2mm (length × width × thickness) and has a inlet 13, and liquid-outlet 14, and liquid-in and out pipes, and a fixing structure 15. Its surface contacted with the bottom unit is a plane without any seal structure. Every pair of inlet and outlet is corresponding to the inlet and outlet areas of each reaction well on the bottom unit. The obtained top unit is marked as B1.
   The top unit with the formation structure
(2) The top unit (Picture 3D) is made from stainless steel plate which can be used repeatedly, with a size as 100mm × 40mm × 2mm (length × width × thickness) and has a inlet and outlet 13 and liquid-in and -out pipes. On its plane contacted with the bottom unit there is the seal structure 16. Its seal structure is an elastic material layer (room temperature self-drying silicone rubber solution, Chengdu Chenguang Research Institute of Chemical Industry) (with a thickness less than 0.1mm) corresponding to the areas outside the reaction wells on the bottom unit. Every pair of inlet and outlet is corresponding to the inlet area and outlet area of each reaction well on the bottom unit. The obtained top unit is marked as B2.

### 3) Examination of characteristics of the analysis-chip with striped-capillary reaction-chamber

PBS is used as a test media in the implementation example. The top and bottom units of the analysis-chip obtained in 1) and 2) mentioned-above are connected with hermetization. This implementation example uses mechanic pressure to complete said seal connection. According to different combinations of the top and bottom units, varied chips are generated (See Table 7). Set the combined object of the top and bottom units upright (inlet on the bottom, outlet on the top). Subject PBS into the entrance of the striped-capillary reaction-chamber. Open the inlet and it can be observed that PBS solution is filling the striped-capillary reaction-chamber through the inlet to the outlet.

### 4) Use of the analysis-chip with the striped-capillary reaction-chamber

Samples used in the implementation example are the same as those in Implementation example 1. During experiment, 4 samples are subjected into the analysis-chip (Table 7) with the striped-capillary reaction-chamber respectively. Each type of sample is subjected into 2 reaction wells respectively. Before the samples are subjected, they are diluted properly.

Two approaches to subjecting sample:
(1) Subjecting sample by batches:
   During operation, sample is subjected into the feeding well and is stopped when it reaches outlet. It will fill the whole striped-capillary reaction-chamber due to capillarity from the inlet to the outlet automatically. There isn't air bubbles observed. Put the analysis-chip into an incubator for reaction for 5 minutes at 37°C.
(2) Subjecting sample continually:
   During operation, sample is warmed up to 37°C. Subject the sample into the feeding well with the flow velocity of 10ul/min at first and 1ul/min later on. The subjecting time is 5 minutes.

After the reaction, the sample is absorbed with paper or drawn out by a pipette via the outlet. Washing solution could be subjected by batches or continually. The total volume subjected is 40ul.

The marker is Rhodamine-labeled goat anti-human secondary antibody (Jackson ImmunoResearch Laboratories). In order to use the label volume as lease as possible, this example uses the batched approach to subject sample. The subject volume is 5ul. Reaction temperature is 37°C. Time for reaction is 5 minutes. The washing after the labeling reaction is the same as that for the sample reaction s. After it is dried, the results are visualized directly with a laser confocal microscope scanner (Afymetric Comp, GMS 418 analysis-chip Scanner). The data are processed to get the results showed in Table 7.

**Table 7**

| Chip | Top unit | Bott om unit | HCV antibody positive serum | | HIV antibody positive serum | | Positive control | | Negative control | |
|---|---|---|---|---|---|---|---|---|---|---|
| | | | HCV antibody | HIV antibody | HCV antibody | HIV antibody | HCV antibody | HIV antibody | HCV antibody | HIV antibody |
| 1 | B1 | A1 | + | - | - | + | + | + | - | - |
| 2 | B1 | A2 | + | - | - | + | + | + | - | - |
| 3 | B1 | A3 | + | - | - | + | + | + | - | - |
| 4 | B1 | A4 | + | - | - | + | + | + | - | - |
| 5 | B1 | A5 | + | - | - | + | + | + | - | - |
| 6 | B2 | A6 | + | - | - | + | + | + | - | - |
| 7 | B2 | A1 | + | - | - | + | + | + | - | - |
| 8 | B2 | A2 | + | - | - | + | + | + | - | - |
| 9 | B2 | A3 | + | - | - | + | + | + | - | - |

| | | | | | | | | | | |
|---|---|---|---|---|---|---|---|---|---|---|
| "+" stands for positive result; "-" negative result | | | | | | | | | | |

### Implementation example 11: Preparation of ligand-ligate analysis-chip and the applications thereof

### 1) Preparation of ligand-ligate chip

The substrate in the implementation example is the epoxy glass slide. Two probes are HBs fusion antigen and anti-HBs antibody (Hepatic Disease Institute, Beijing People Hospital).

The preparation of the monoplane analysis-chip with the striped-capillary reaction-chamber is the same as that in Implementation example 9. Though ligand and ligate in the invention could be in varied fashions and immobilized onto different corresponding positions, in the example, the 3 x 3 arrays of HBs fusion antigen are immobilized near the inlet of each striped-capillary reaction-chamber; however, a 3 x 3 arrays of anti-HBs antibody are immobilized near the outlet.

### 2) Examination of characteristics of the capillary chip

The method is the same as that in Implementation example 9

### 3) Application of the ligand-ligate chip

The samples used in this implementation example are HBs antigen positive human serum, HBs antigen negative human serum and HBs antibody positive human serum. All the samples are identified by a pre-analysis with the classical analysis-chip with the open mono-reactor under the same reactive condition. During experiment, 3 samples are placed into the prepared analysis-chip mentioned-above respectively. This example uses the batch-approach subject sample in Implementation example 9. The test method is the same as that in Implementation example 9. The data are processed to get the results showed in Table 8.

**Table 8**

| Multiple of sample dilution | HBs antigen positive serum | HBs antigen negative serum | HBs antibody positive serum |
|---|---|---|---|
| 10 | + | - | + |
| 100 | + | - | + |

| | | | |
|---|---|---|---|
| "+" stands for positive result; "-" negative result. | | | |

## Claims

1. An analysis-chip, which comprises one or more flow or non-flow reactors with minimal-height, wherein said reactor comprises at least one or more striped-capillary reaction-chambers (1) with a height of less than 1000 µm, wherein said reaction-chamber (1) comprises:
1) top plane (5) and bottom plane (6) with a width more than 600 µm;
2) probe-ligand and substrate probe-region (7) in which said probe-ligand is immobilized;
3) closed partition structure (8) with a height of 1-1000 µm; and
4) inlet (9) and outlet (10),
and wherein:
(i) said top plane (5) and bottom plane (6) are parts of top unit (11) and bottom unit (12) of said striped-capillary reaction-chamber(1), respectively;
(ii) said substrate probe-region (7) is on said top plane (5) or/and bottom plane (6);
(iii) said closed partition structure (8) is placed between said top plane (5) and bottom plane (6);
(iv) material and dimension of said planes, and distance between said top plane (5) and bottom plane (6) are such that the capillary phenomenon of analysis media can take place in said chamber (1); and
(v) said substrate probe-region (7) presents a water-absorptivity less than 0.1 g/g and a water contact angle as 40-80°.

2. The analysis-chip of claim 1, wherein said striped-capillary reaction-chambers (1) present a height of less than 500 µm.

3. The analysis-chip of claim 1, wherein:
(i) in said striped-capillary reaction-chamber: i). said closed partition-structure (8) presents a height of 1-300 µm ; ii). said top plane (5) presents a width of 1000-15000 µm and a length more than 1000 µm; and iii) said bottom plane (6) presents a width of 1000-15000 µm and a length more than 1000 µm; and/or
(ii) said reactor comprises reactor-protecting structure comprising protective unit with a distance less than 1000 µm, wherein said protective unit closes at least partially said reactor structure when no sample is subjected, and it is irreversibly removed completely or partially when sample is to be subjected.

4. The analysis-chip of claim 3, wherein said closed partition structure (8) presents a height of 30-100 µm.

5. The analysis-chip of claim 3, wherein said top plane (5) and bottom plane (6) in said striped-capillary reaction-chamber presents a width of 1500-15000 µm.

6. The analysis-chip of claim 3, wherein said top plane (5) and bottom plane (6) in said striped-capillary reaction-chamber presents a width of 2500-15000 µm.

7. The analysis-chip of claim 3, wherein said closed partition structure (8) includes one or more of the following reversible or irreversible enclosing structures:
(i) thermal enclosing structure;
(ii) chemical enclosing structure;
(iii) reversible or irreversible adhesive layer
(iv) highly-hydrophobic layer; and
(v) mechanic enclosing unit including coating, plate or tape of elastic polymer.

8. The analysis-chip of claim 3, wherein said top unit (11) or/and bottom unit (12) of said reaction-chamber(1) present a thickness less than 1 mm, and a detecting-light transparency rate more than 90%.

9. The analysis-chip of claim 3, wherein said top unit (11) or/and bottom unit (12) of said reaction-chamber(1) present a thickness less than 0.2 mm, and a detecting-light transparency rate more than 90%.

10. The analysis-chip of claim 3, wherein said probe-ligand is immobilized on either said top plane (5) or said bottom plane (6), wherein said plane with said immobilized probe-ligand presents hydrophilic property whereas said plane without said immobilized probe-ligand presents hydrophobic property.

11. The analysis-chip of claim 3, wherein said top plane (5) is made of glass material whereas said bottom plane (6) is made of hydrophilic or hydrophobic plastic.

12. The analysis-chip of claim 3, wherein one or more said probe-ligands are immobilized in one area whereas one or more ligates of said ligands are immobilized in one other area in said probe region.

13. The analysis-chip of claim 12, wherein said ligand and ligate include antigen and antibody.

14. The analysis-chip of claim 3, wherein said protective unit presents a distance less than 1000 µm from said substrate probe-region.

15. The analysis-chip of claim 3, wherein said protective unit presents a distance less than 500 µm from said substrate probe-region.

16. The analysis-chip of claim 3, wherein said protecting unit includes one or more of the following: organic film or/and plate, film or/and plate of metal-organic complex, and slide.

17. The analysis-chip of claim 16, wherein said protecting unit is connected with the reactor through one or more of the following reversible/ irreversible enclosing structures:
(i) thermal enclosing structure;
(ii) chemical enclosing structure; and
(iii) reversible or irreversible adhesive layer.

18. The analysis-chip of claim 16, wherein wherein said protecting unit is precut for the convenience of possible removal.

## Patentansprüche

1. Analysechip umfassend ein oder mehrere durchströmte oder nicht durchströmte Reaktoren mit minimaler Höhe, wobei der Reaktor wenigstens eine oder mehrere Streifenkapillar-Reaktionskammern (1) mit einer Höhe von weniger als 1000 µm umfasst, wobei die Reaktionskammer (1) umfasst:
1) obere Ebene (5) und untere Ebene (6) mit einer Breite von mehr als 600 µm;
2) Sondenligand und Substratsondenregion (7) in welcher der Sondenligand immobilisiert ist;
3) Struktur (8) mit geschlossener Abtrennung mit einer Höhe von 1-1000 µm; und
4) Einlass (9) und Auslass (10),
und wobei
(i) die obere Ebene und die untere Ebene (6) Teile der oberen Einheit (11) beziehungsweise der unteren Einheit (12) der Streifenkapillar-Reaktionskammer (1) sind;
(ii) der Substratsondenbereich (7) auf der oberen Ebene (5) oder/und auf der unteren Ebene (6) angeordnet ist;
(iii) die Struktur (8) mit geschlossener Abtrennung zwischen der oberen Ebene (5) und der unteren Ebene (6) angeordnet ist;
(iv) Material und Abmessungen der Ebenen und der Abstand zwischen der oberen Ebene (5) und der unteren Ebene (6) so sind, dass das Kapillarphänomen des Analysemediums in der Kammer (1) stattfinden kann; und
(v) der Substratsondenbereich (7) ein Wasserabsorptionsvermögen von weniger als 0,1 g/g und einen Wasserkontaktwinkel von 40-80° aufweist.

2. Analysechip nach Anspruch 1, wobei die Streifenkapillar-Reaktionskammern (1) eine Höhe von weniger als 500 µm aufweisen.

3. Analysechip nach Anspruch 1, wobei:
(i) in der Streifenkapillar-Reaktionskammer: i) die Struktur (8) mit geschlossener Abtrennung eine Höhe von 1-300µm aufweist; ii) die obere Ebene eine Breite von 1000-15000µm und eine Länge von mehr als 1000µm aufweist; und/oder
(ii) der Reaktor eine Reaktorschutzstruktur umfasst, umfassend eine Schutzeinheit mit einem Abstand von weniger als 1000µm, wobei die Schutzeinheit die Reaktorstruktur wenigstens teilweise schließt, wenn keine Probe unterworfen ist, und vollständig oder teilweise irreversibel entfernt ist, wenn eine Probe unterworfen werden soll.

4. Analysechip nach Anspruch 3, wobei die Struktur (8) mit geschlossener Abtrennung eine Höhe von 30-100µm aufweist.

5. Analysechip nach Anspruch 3, wobei die obere Ebene (5) und die untere Ebene (6) in der Streifenkapillar-Reaktionskammer eine Breite von 1500-15000 µm aufweist.

6. Analysechip nach Anspruch 3, wobei die obere Ebene (5) und die untere Ebene (6) in der Streifenkapillar-Reaktionskammer eine Breite von 2500-15000 µm aufweist.

7. Analysechip nach Anspruch 3, wobei die Struktur (8) mit geschlossener Abtrennung ein oder mehrere der folgenden reversibel oder irreversibel einschließenden Strukturen enthält:
(i) thermisch einschließende Struktur;
(ii) chemisch einschließende Struktur
(iii) reversible oder irreversible Haftschicht;
(iv) stark hydrophobe Schicht; und
(v) mechanisch einschließende Einheit, einschließlich Beschichtung, Platte oder Band aus elastischem Polymer.

8. Analysechip nach Anspruch 3, wobei die obere Einheit (11) oder/und die untere Einheit (12) der Reaktionskammer eine Dicke von weniger als 1 mm, und eine Detektionslicht-Transparenzrate von mehr als 90 % aufweist.

9. Analysechip nach Anspruch 3, wobei die obere Einheit (11) und/oder die untere Einheit (12) der Reaktionskammer eine Dicke von weniger als 0,2 mm, und eine Detektionslicht-Transparenzrate von mehr als 90 % aufweist.

10. Analysechip nach Anspruch 3, wobei der Sondenligand entweder auf der oberen Ebene (5) oder der unteren Ebene (6) immobilisiert ist, wobei die Ebene mit dem immobilisierten Sondenliganden hydrophile Eigenschaften aufweist, wohingegen die Ebene ohne immobilisierten Sondenliganden hydrophobe Eigenschaften aufweist.

11. Analysechip nach Anspruch 3, wobei die obere Ebene (5) aus einem Glassmaterial hergestellt ist, wohingegen die untere Ebene (6) aus einem hydrophilen oder hydrophoben Kunststoff hergestellt ist.

12. Analysechip nach Anspruch 3, wobei ein oder mehrere der Sondenliganden in einem Bereich immobilisiert sind, wohingegen ein oder mehrere Ligaten dieser Liganden in einem anderen Bereich der Sondenregion immobilisiert sind.

13. Analysechip nach Anspruch 12, wobei der Ligand und der Ligat Antigen und Antikörper enthalten.

14. Analysechip nach Anspruch 3, wobei die Schutzeinheit einen Abstand von weniger als 1000 µm von der Substratsondenregion aufweist.

15. Analysechip nach Anspruch 3, wobei die Schutzeinheit einen Abstand von weniger als 500 µm von der Substratsondenregion aufweist.

16. Analysechip nach Anspruch 3, wobei die Schutzeinheit ein oder mehrere der folgenden aufweist: organische Schicht oder/und Platte, Schicht oder/und Platte aus metallorganischem Komplex und Folie (slide).

17. Analysechip nach Anspruch 16, wobei die Schutzeinheit mit dem Reaktor durch eine oder mehrere der folgenden reversibel / irreversibel einschließenden Strukturen verbunden ist:
(i) thermisch einschließende Struktur;
(ii) chemisch einschließende Struktur; und
(iii) reversible oder irreversible Haftschicht.

18. Analysechip nach Anspruch 16, wobei die Schutzeinheit vorgeschnitten ist, um die mögliche Entfernung zu vereinfachen.

## Revendications

1. Puce d'analyse, laquelle comprend un ou plusieurs réacteurs avec écoulement ou sans écoulement présentant une hauteur minimale, dans laquelle ledit réacteur comprend au moins une ou plusieurs chambres réactionnelle capillaire en forme de bande (1) dont la hauteur est inférieure à 1 000 µm, dans laquelle ladite chambre réactionnelle (1) comprend :
1) un plan supérieur (5) et un plan inférieur (6) dont la largeur est supérieure à 600 µm ;
2) un ligand-sonde et une région-sonde de substrat (7) dans laquelle ledit ligand-sonde est immobilisé ;
3) une structure de séparation fermée (8) dont la hauteur est de 1 à 1 000 µm ; et
4) une entrée (9) et une sortie (10),
et dans laquelle :
(i) lesdits plan supérieur (5) et plan inférieur (6) sont des parties d'une unité supérieure (11) et d'une unité inférieure (12) de ladite chambre réactionnelle capillaire en forme de bande (1), respectivement ;
(ii) ladite région-sonde de substrat (7) est sur ledit plan supérieur (5) et/ou plan inférieur (6) ;
(iii) ladite structure de séparation fermée (8) est placée entre lesdits plan supérieur (5) et plan inférieur (6) ;
(iv) le matériau et la dimension desdits plans, et la distance entre lesdits plan supérieur (5) et plan inférieur (6) sont tels que le phénomène capillaire des milieux d'analyse peut avoir lieu dans ladite chambre (1) ; et
(v) ladite région-sonde de substrat (7) présente une capacité d'absorption d'eau inférieure à 0,1 g/g et un angle de contact avec l'eau de 40 à 80°.

2. Puce d'analyse selon la revendication 1, dans laquelle lesdites chambres réactionnelles capillaires en forme de bande (1) présentent une hauteur inférieure à 500 µm.

3. Puce d'analyse selon la revendication 1, dans laquelle :
(i) dans ladite chambre réactionnelle capillaire en forme de bande : i) ladite structure de séparation fermée (8) présente une hauteur de 1 à 300 µm ; ii) ledit plan supérieur (5) présente une largeur de 1 000 à 15 000 µm et une longueur supérieure à 1 000 µm ; et iii) ledit plan inférieur (6) présente une largeur de 1 000 à 15 000 µm et une longueur supérieure à 1 000 µm ; et/ou
(ii) ledit réacteur comprend une structure de protection de réacteur comprenant une unité protectrice à une distance inférieure à 1 000 µm, ladite unité protectrice fermant au moins partiellement ladite structure de réacteur lorsque aucun échantillon n'est soumis, et elle est entièrement ou partiellement retirée de manière irréversible lorsque l'échantillon doit être soumis.

4. Puce d'analyse selon la revendication 3, dans laquelle ladite structure de séparation fermée (8) présente une hauteur de 30 à 100 µm.

5. Puce d'analyse selon la revendication 3, dans laquelle lesdits plan supérieur (5) et plan inférieur (6) dans ladite chambre réactionnelle capillaire en forme de bande présentent une largeur de 1 500 à 15 000 µm.

6. Puce d'analyse selon la revendication 3, dans laquelle lesdits plan supérieur (5) et plan inférieur (6) dans ladite chambre réactionnelle capillaire en forme de bande présentent une largeur de 2 500 à 15 000 µm.

7. Puce d'analyse selon la revendication 3, dans laquelle ladite structure de séparation fermée (8) inclut une ou plusieurs des structures d'enceinte réversibles ou irréversibles suivantes :
(i) une structure d'enceinte thermique ;
(ii) une structure d'enceinte chimique ;
(iii) une couche adhésive réversible ou irréversible ;
(iv) une couche fortement hydrophobe ; et
(v) une unité d'enceinte mécanique incluant un revêtement, une plaque ou une bande de polymère élastique.

8. Puce d'analyse selon la revendication 3, dans laquelle ladite unité supérieure (11) et/ou unité inférieure (12) de ladite chambre réactionnelle (1) présente une épaisseur inférieure à 1 mm, et un taux de transparence de la lumière de détection supérieur à 90 %.

9. Puce d'analyse selon la revendication 3, dans laquelle ladite unité supérieure (11) et/ou unité inférieure (12) de ladite chambre réactionnelle (1) présente une épaisseur inférieure à 0,2 mm, et un taux de transparence de la lumière de détection supérieur à 90 %.

10. Puce d'analyse selon la revendication 3, dans laquelle ledit ligand-sonde est immobilisé soit sur ledit plan supérieur (5) soit sur ledit plan inférieur (6), dans laquelle ledit plan avec ledit ligand-sonde immobilisé présente des propriétés hydrophiles alors que ledit plan sans ledit ligand-sonde immobilisé présente des propriétés hydrophobes.

11. Puce d'analyse selon la revendication 3, dans laquelle ledit plan supérieur (5) est constitué d'une matière de verre alors que ledit plan inférieur (6) est constitué d'une matière plastique hydrophile ou hydrophobe.

12. Puce d'analyse selon la revendication 3, dans laquelle un ou plusieurs desdits ligands-sondes sont immobilisés dans une zone alors qu'un ou plusieurs ligats desdits ligands sont immobilisés dans une autre zone dans ladite région-sonde.

13. Puce d'analyse selon la revendication 12, dans laquelle lesdits ligand et ligat incluent un antigène et un anticorps.

14. Puce d'analyse selon la revendication 3, dans laquelle ladite unité protectrice présente une distance inférieure à 1 000 µm par rapport à ladite région-sonde de substrat.

15. Puce d'analyse selon la revendication 3, dans laquelle ladite unité protectrice présente une distance inférieure à 500 µm par rapport à ladite région-sonde de substrat.

16. Puce d'analyse selon la revendication 3, dans laquelle ladite unité protectrice inclut un ou plusieurs des éléments suivants : film et/ou plaque organique, film et/ou plaque en complexe métal-organique, et lamelle.

17. Puce d'analyse selon la revendication 16, dans laquelle ladite unité protectrice est reliée au réacteur par une ou plusieurs des structures d'enceinte réversibles/irréversibles suivantes :
(i) une structure d'enceinte thermique ;
(ii) une structure d'enceinte chimique ; et
(iii) une couche adhésive réversible ou irréversible.

18. Puce d'analyse selon la revendication 16, dans laquelle ladite unité protectrice est prédécoupée pour faciliter un retrait éventuel.
